# EUROPEAN PATENT APPLICATION

(11) **EP 3 733 836 A1**
(43) Date of publication of application: **04.11.2020**
(21) Application number: 18894130.6
(22) Date of filing: 27.12.2018
(51) Int. Cl.: C12N 5/071, C12M 3/00, C12N 15/09

(54) **CELL AGGREGATION INHIBITOR**

(30) Priority: 28.12.2017 JP 2017253147
(71) Applicant: Kaneka Corporation, Osaka-shi, Osaka 530-8288 (JP)
(72) Inventor: IBUKI Masato, Kobe-shi, Hyogo 650-0047 (JP)
(74) Representative: Hoffmann Eitle
(86) International application number: PCT/JP2018/048313
(87) International publication number: WO 2019/131941

(57) **Abstract**

Provided is a means for appropriately controlling the size of cell aggregates without relying on mechanical/physical means. The present invention relates to a cell aggregation suppressor for use in suspension culture of cells, comprising an agonist of thrombin receptor. The present invention also relates to a method for producing cell aggregates, comprising a step of culturing cells in suspension in a culture medium comprising an agonist of thrombin receptor.

SEQUENCE LISTING

<110>  Kaneka Corporation

<120>  An agent for inhibiting cell aggregation

<130>  B170688

<150>  JP 2017-253147
<151>  2017-12-28

<160>  22

<170>  PatentIn version 3.5

<210>  1
<211>  5
<212>  PRT
<213>  Artificial

<220>
<223>  synthetic

<400>  1

Ser Phe Leu Leu Arg
1               5


<210>  2
<211>  5
<212>  PRT
<213>  Artificial

<220>
<223>  synthetic

<400>  2

Ser Phe Phe Leu Arg
1               5


<210>  3
<211>  6
<212>  PRT
<213>  Artificial

<220>
<223>  synthetic

<400>  3

Thr Phe Arg Gly Ala Pro
1               5


<210>  4
<211>  7
<212>  PRT
<213>  Artificial

<220>

<223>  synthetic

<400>  4

Ser Phe Gly Asx Gly Gly Pro
1               5


<210>  5
<211>  6
<212>  PRT
<213>  Artificial

<220>
<223>  synthetic

<400>  5

Gly Tyr Pro Gly Gln Val
1               5


<210>  6
<211>  6
<212>  PRT
<213>  Artificial

<220>
<223>  synthetic

<400>  6

Gly Tyr Pro Gly Phe Lys
1               5


<210>  7
<211>  6
<212>  PRT
<213>  Artificial

<220>
<223>  synthetic

<400>  7

Ser Phe Leu Leu Arg Asn
1               5


<210>  8
<211>  6
<212>  PRT
<213>  Artificial

<220>
<223>  synthetic

<400>  8

Ser Phe Leu Leu Arg Asn
1               5

<210>  9
<211>  10
<212>  PRT
<213>  Artificial

<220>
<223>  synthetic

<400>  9

Thr Phe Leu Leu Arg Asn Pro Asn Asp Lys
1               5                   10


<210>  10
<211>  6
<212>  PRT
<213>  Artificial

<220>
<223>  synthetic

<400>  10

Phe Ser Leu Leu Arg Asn
1               5


<210>  11
<211>  5
<212>  PRT
<213>  Artificial

<220>
<223>  synthetic

<400>  11

Phe Leu Leu Arg Asn
1               5


<210>  12
<211>  6
<212>  PRT
<213>  Artificial

<220>
<223>  synthetic

<400>  12

Ser Leu Ile Gly Lys Val
1               5


<210>  13
<211>  6
<212>  PRT
<213>  Artificial

<220>

<223>  synthetic

<400>  13

Ser Leu Ile Gly Arg Leu
1               5


<210>  14
<211>  6
<212>  PRT
<213>  Artificial

<220>
<223>  synthetic

<400>  14

Ala Tyr Pro Gly Lys Phe
1               5


<210>  15
<211>  21
<212>  DNA
<213>  Artificial

<220>
<223>  primer

<400>  15
cctcatgaag atcctcaccg a                                                21


<210>  16
<211>  21
<212>  DNA
<213>  Artificial

<220>
<223>  primer

<400>  16
ttgccaatgg tgatgacctg g                                                21


<210>  17
<211>  20
<212>  DNA
<213>  Artificial

<220>
<223>  primer

<400>  17
gaagtcccgg gctttgttcc                                                  20


<210>  18
<211>  21
<212>  DNA
<213>  Artificial

<220>
<223>  primer

<400>  18
tggcactcag aggaagcgta a                                                21


<210>  19
<211>  21
<212>  DNA
<213>  Artificial

<220>
<223>  primer

<400>  19
ggccctcaga gatgatcagt c                                                21


<210>  20
<211>  22
<212>  DNA
<213>  Artificial

<220>
<223>  primer

<400>  20
gtctcgaact cctgacctca ag                                               22


<210>  21
<211>  22
<212>  DNA
<213>  Artificial

<220>
<223>  primer

<400>  21
caccaatccc atccacactc ac                                               22


<210>  22
<211>  21
<212>  DNA
<213>  Artificial

<220>
<223>  primer

<400>  22
gcaaagctcc taccgtacca c                                                21

## Description

### Technical Field

The present invention relates to cell aggregation suppressors and methods for suppressing aggregation of cells. The present invention also relates to methods for producing cell aggregates and cell aggregates produced thereby. The present invention also relates to cell culture compositions and cell culture media.

### Background Art

Recent research on human pluripotent stem cells (e.g., human ES cells and human iPS cells) has increasingly made regenerative medicine come in reality. These cells possess an ability to proliferate infinitely and an ability to differentiate into various types of cells. Thus, regenerative medicine using the pluripotent stem cells should radically change therapeutic interventions against, for example, refractory diseases and lifestyle-related diseases. It has already been possible that the pluripotent stem cells can be induced and differentiated *in vitro* into various types of cells including neurons, cardiomyocytes, blood cells, and retinal cells.

One of objectives directed toward practical use of regenerative medicine in which pluripotent stem cells are used to regenerate a variety of organs involves how a large number of cells necessary for regeneration of organs can be produced efficiently. For example, the regeneration of a liver requires about 2 × 10¹¹ cells. A substrate plate with an area of 10⁶ cm² or more is needed so as to culture the above number of cells using adherent culture on a flat substrate plate. This means that about 20,000 common 10-cm dishes are needed. Because the number of cells to be obtained using adherent culture on a surface of the substrate plate depends on the surface area of the culture plate, it is difficult to scale up the culture. Accordingly, it is hard to provide an enough number of cells to make regenerative medicine available.

Here, it is easy to scale up suspension culture in which cells are cultured in suspension in a liquid culture medium. Hence, the suspension culture should be fit for mass production of cells.

For example, Non-Patent Literature 1 discloses a process for producing spheroids with a uniform size, the process comprising: using a spinner flask as cell cultureware for suspension culture; and culturing human pluripotent stem cells in suspension while strongly stirring a liquid culture medium.

Non-Patent Literature 2 discloses a process for producing spheroids with a uniform size in each micro-well, the process comprising using a substrate plate on which small micro-wells are formed.

Non-Patent Literature 3 discloses a culturing method comprising: using a culture medium the viscosity and specific gravity of which is adjusted; keeping pluripotent stem cells in suspension; and reducing a collision between the cells.

Patent Literature 1 discloses a technology in which cells are cultured while being subjected to rotary shaking culture in a liquid culture medium, so that cell aggregates are produced.

Patent Literature 2 discloses a method in which pluripotent stem cells are cultured in suspension until the average diameter of cell aggregates reaches about 200 to 300 µm.

Patent Literature 3 discloses a technique for suppressing cell aggregation by culturing cells in suspension in a culture medium containing lysophospholipids such as lysophosphatidic acid (LPA) and sphingosine-1-phosphate (SIP).

### Citation List

### Patent Literature

Patent Literature 1: JP Patent Publication (Kokai) No. 2003-304866A
Patent Literature 2: WO2013/077423A
Patent Literature 3: WO2016/121737A

### Non-Patent Literature

Non-Patent Literature 1: Olmer R. et al., Tissue Engineering: Part C, Volume 18 (10): 772-784 (2012)
Non-Patent Literature 2: Ungrin MD et al., PLoS ONE, 2008, 3(2), e1565
Non-Patent Literature 3: Otsuji GT et al., Stem Cell Reports, Volume 2: 734-745 (2014)

### Summary of Invention

### Technical Problem

The present inventors have found that non-specific adsorption of membrane proteins and cell membranes between cells, and adhesion between cells mediated by cadherin on the cell surface are important mechanisms in culturing adhesive cells such as pluripotent stem cells in suspension. That is, a challenge in the technique of suspension culture is to produce cell aggregates with an appropriate size without damaging the bindings of membrane proteins of the cells, cadherins on the cell surface, or the like. However, the techniques of suspension culture disclosed in Non-Patent Literatures 1 to 3 and Patent Literatures 1 or 2 had the following problems:

The process of Non-Patent Literature 1 likely causes cells to die due to shear stress, which is a defect of the process.

In the process of Non-Patent Literature 2, it is difficult to scale up a culture and to change a culture medium.

In the method of Non-Patent Literature 3, because of less movement of a culture medium during culture, oxygen and nutritional components are less likely to be supplied to cell aggregates.

Patent Literature 1 fails to disclose a means for controlling the size of cell aggregates to an appropriate size.

Patent Literature 2 discloses adding, to a culture medium, a water-soluble polymer as a means for preventing adhesion between cell aggregates, so that the viscosity increases. This causes the same defect as in the case of Non-Patent Literature 3, in which oxygen and nutritional components are less likely to be supplied to cell aggregates.

In order to solve the above problems, the inventors have developed a technique for suspension culture that can suppress cell aggregation by culturing cells in suspension in a culture medium containing lysophospholipids in Patent Literature 3 and produce aggregates with an appropriate size without damaging the cells.

However, Patent Literature 3 discloses only lysophospholipids as components that suppress cell aggregation. The present inventors have contemplated that the culture medium in suspension culture provided with components that suppress cell aggregation in addition to lysophospholipids would allow the size of cell aggregates to be appropriately controlled without relying on mechanical/physical means.

### Solution to Problem

As a result of extensive researches, the present inventors have found that agonists of a thrombin receptor exhibit an action of suppressing aggregation of cells by being present in a culture medium in suspension culture, and have completed the following present invention.
(1) A cell aggregation suppressor for use in suspension culture of cells, comprising an agonist of a thrombin receptor.
(2) The cell aggregation suppressor according to item (1), wherein a concentration of the agonist of a thrombin receptor is 7.4 µg/mL or more and 3.8 mg/mL or less.
(3) The cell aggregation suppressor according to item (1) or (2), wherein the thrombin receptor is at least one selected from the group consisting of PAR-1, PAR-2, PAR-3, and PAR-4.
(4) The cell aggregation suppressor according to any one of items (1) to (3), wherein the agonist of a thrombin receptor is any one of peptides (a), (b), and (c):
   (a) a peptide consisting of an amino acid sequence of SEQ ID NO: 1, 2, 7, or 8;
   (b) a peptide consisting of an amino acid sequence derived from the amino acid sequence of SEQ ID NO: 1, 2, 7, or 8 by substitution, deletion, insertion, and/or addition of one or two amino acids;
   (c) a peptide consisting of an amino acid sequence having 60% or more sequence identity to the amino acid sequence of SEQ ID NO: 1, 2, 7, or 8.
(5) The cell aggregation suppressor according to any one of items (1) to (3), wherein the agonist of a thrombin receptor is any one of peptides (a), (b), and (c):
   (a) a peptide comprising an amino acid sequence of SEQ ID NO: 1, 2, 7, or 8;
   (b) a peptide comprising an amino acid sequence derived from the amino acid sequence of SEQ ID NO: 1, 2, 7, or 8 by substitution, deletion, insertion, and/or addition of one or two amino acids;
   (c) a peptide comprising an amino acid sequence having 60% or more sequence identity to the amino acid sequence of SEQ ID NO: 1, 2, 7, or 8.
(6) The cell aggregation suppressor according to any one of items (1) to (5), wherein the cells are stem cells.
(7) A method for producing cell aggregates, comprising a step of culturing cells in suspension in a culture medium comprising an agonist of a thrombin receptor.
(8) The method according to item (7), wherein a concentration of the agonist of a thrombin receptor in the culture medium is 3.7 ng/mL or more and 3.8 mg/mL or less.
(9) The method according to item (7) or (8), wherein the thrombin receptor is at least one selected from the group consisting of PAR-1, PAR-2, PAR-3, and PAR-4.
(10) The method according to any one of items (7) to (9), wherein the agonist of a thrombin receptor is any one of peptides (a), (b), and (c):
   (a) a peptide consisting of an amino acid sequence of SEQ ID NO: 1, 2, 7, or 8;
   (b) a peptide consisting of an amino acid sequence derived from the amino acid sequence of SEQ ID NO: 1, 2, 7, or 8 by substitution, deletion, insertion, and/or addition of one or two amino acids;
   (c) a peptide consisting of an amino acid sequence having 60% or more sequence identity to the amino acid sequence of SEQ ID NO: 1, 2, 7, or 8.
(11) The method according to any one of items (7) to (9), wherein the agonist of a thrombin receptor is any one of peptides (a), (b), and (c):
   (a) a peptide comprising an amino acid sequence of SEQ ID NO: 1, 2, 7, or 8;
   (b) a peptide comprising an amino acid sequence derived from the amino acid sequence of SEQ ID NO: 1, 2, 7, or 8 by substitution, deletion, insertion, and/or addition of one or two amino acids;
   (c) a peptide comprising an amino acid sequence having 60% or more sequence identity to the amino acid sequence of SEQ ID NO: 1, 2, 7, or 8.
(12) The method according to any one of items (7) to (11), wherein the cells are stem cells.
(13) A cell aggregate obtained by the method according to any one of items (7) to (12).
(14) A cell culture composition comprising cells, a culture medium, and an agonist of a thrombin receptor.
(15) The cell culture composition according to item (14), wherein a concentration of the agonist of a thrombin receptor is 3.7 ng/mL or more and 3.8 mg/mL or less.
(16) The cell culture composition according to item (14) or (15), wherein the thrombin receptor is at least one selected from the group consisting of PAR-1, PAR-2, PAR-3, and PAR-4.
(17) The cell culture composition according to any one of items (14) to (16), wherein the agonist of a thrombin receptor is any one of peptides (a), (b), and (c):
   (a) a peptide consisting of an amino acid sequence of SEQ ID NO: 1, 2, 7, or 8;
   (b) a peptide consisting of an amino acid sequence derived from the amino acid sequence of SEQ ID NO: 1, 2, 7, or 8 by substitution, deletion, insertion, and/or addition of one or two amino acids;
   (c) a peptide consisting of an amino acid sequence having 60% or more sequence identity to the amino acid sequence of SEQ ID NO: 1, 2, 7, or 8.
(18) The cell culture composition according to any one of items (14) to (16), wherein the agonist of a thrombin receptor is any one of peptides (a), (b), and (c):
   (a) a peptide comprising an amino acid sequence of SEQ ID NO: 1, 2, 7, or 8;
   (b) a peptide comprising an amino acid sequence derived from the amino acid sequence of SEQ ID NO: 1, 2, 7, or 8 by substitution, deletion, insertion, and/or addition of one or two amino acids;
   (c) a peptide comprising an amino acid sequence having 60% or more sequence identity to the amino acid sequence of SEQ ID NO: 1, 2, 7, or 8.
(19) The cell culture composition according to any one of items (14) to (18), wherein the cells are stem cells.
(20) The cell culture composition according to any one of items (14) to (19), wherein the cells are in a form of cell aggregates.
(21) A method for suppressing aggregation of cells, comprising a step of culturing the cells in suspension in a culture medium comprising an agonist of a thrombin receptor.
(22) The method according to item (21), wherein the concentration of the agonist of a thrombin receptor in the culture medium is 3.7 ng/mL or more and 3.8 mg/mL or less.
(23) The method according to item (21) or (22), wherein the thrombin receptor is at least one selected from the group consisting of PAR-1, PAR-2, PAR-3, and PAR-4.
(24) The method according to any one of items (21) to (23), wherein the agonist of a thrombin receptor is any one of peptides (a), (b), and (c):
   (a) a peptide consisting of an amino acid sequence of SEQ ID NO: 1, 2, 7, or 8;
   (b) a peptide consisting of an amino acid sequence derived from the amino acid sequence of SEQ ID NO: 1, 2, 7, or 8 by substitution, deletion, insertion, and/or addition of one or two amino acids;
   (c) a peptide consisting of an amino acid sequence having 60% or more sequence identity to the amino acid sequence of SEQ ID NO: 1, 2, 7, or 8.
(25) The method according to any one of items (21) to (23), wherein the agonist of a thrombin receptor is any one of peptides (a), (b), and (c):
   (a) a peptide comprising an amino acid sequence of SEQ ID NO: 1, 2, 7, or 8;
   (b) a peptide comprising an amino acid sequence derived from the amino acid sequence of SEQ ID NO: 1, 2, 7, or 8 by substitution, deletion, insertion, and/or addition of one or two amino acids;
   (c) a peptide comprising an amino acid sequence having 60% or more sequence identity to the amino acid sequence of SEQ ID NO: 1, 2, 7, or 8.
(26) The method according to any one of items (21) to (25), wherein the cells are stem cells.
(27) A cell culture medium comprising a culture medium and an agonist of a thrombin receptor.
(28) The cell culture medium according to item (27), wherein a concentration of the agonist of a thrombin receptor is 3.7 ng/mL or more and 3.8 mg/mL or less.
(29) The cell culture medium according to item (27) or (28), wherein the thrombin receptor is at least one selected from the group consisting of PAR-1, PAR-2, PAR-3, and PAR-4.
(30) The cell culture medium according to any one of items (27) to (29), wherein the agonist of a thrombin receptor is any one of peptides (a), (b), and (c):
   (a) a peptide consisting of an amino acid sequence of SEQ ID NO: 1, 2, 7, or 8;
   (b) a peptide consisting of an amino acid sequence derived from the amino acid sequence of SEQ ID NO: 1, 2, 7, or 8 by substitution, deletion, insertion, and/or addition of one or two amino acids;
   (c) a peptide consisting of an amino acid sequence having 60% or more sequence identity to the amino acid sequence of SEQ ID NO: 1, 2, 7, or 8.
(31) The cell culture medium according to any one of items (27) to (29), wherein the agonist of a thrombin receptor is any one of peptides (a), (b), and (c):
   (a) a peptide comprising an amino acid sequence of SEQ ID NO: 1, 2, 7, or 8;
   (b) a peptide comprising an amino acid sequence derived from the amino acid sequence of SEQ ID NO: 1, 2, 7, or 8 by substitution, deletion, insertion, and/or addition of one or two amino acids;
   (c) a peptide comprising an amino acid sequence having 60% or more sequence identity to the amino acid sequence of SEQ ID NO: 1, 2, 7, or 8.
(32) The cell culture medium according to any one of items (27) to (31), further comprising a growth factor.
(33) The cell culture medium according to any one of items (27) to (32) for use in culture of stem cells.
(34) The cell culture medium according to any one of items (27) to (33) for producing cell aggregates.
(35) The cell aggregation suppressor according to any one of items (1) to (6), the method according to any one of items (7) to (12), the cell aggregate according to item (13), the cell culture composition according to any one of items (14) to (20), the method according to any one of items (21) to (26), or the cell culture medium according to any one of items (27) to (34), wherein the agonist of a thrombin receptor is any one of peptides (a), (b), and (c):
   (a) a peptide consisting of an amino acid sequence of SEQ ID NO: 1, 2, 3, 4, 5, 6, 7, 8, 9, 10, 11, 12, 13, or 14;
   (b) a peptide consisting of an amino acid sequence derived from the amino acid sequence of SEQ ID NO: 1, 2, 3, 4, 5, 6, 7, 8, 9, 10, 11, 12, 13, or 14 by substitution, deletion, insertion, and/or addition of one or two amino acids;
   (c) a peptide consisting of an amino acid sequence having 60% or more sequence identity to the amino acid sequence of SEQ ID NO: 1, 2, 3, 4, 5, 6, 7, 8, 9, 10, 11, 12, 13, or 14.
(36) The cell aggregation suppressor according to any one of items (1) to (6), the method according to any one of items (7) to (12), the cell aggregate according to item (13), the cell culture composition according to any one of items (14) to (20), the method according to any one of items (21) to (26), or the cell culture medium according to any one of items (27) to (34), wherein the agonist of a thrombin receptor is any one of peptides (a), (b), and (c):
   (a) a peptide comprising an amino acid sequence of SEQ ID NO: 1, 2, 3, 4, 5, 6, 7, 8, 9, 10, 11, 12, 13, or 14;
   (b) a peptide comprising an amino acid sequence derived from the amino acid sequence of SEQ ID NO: 1, 2, 3, 4, 5, 6, 7, 8, 9, 10, 11, 12, 13, or 14 by substitution, deletion, insertion, and/or addition of one or two amino acids;
   (c) a peptide comprising an amino acid sequence having 60% or more sequence identity to the amino acid sequence of SEQ ID NO: 1, 2, 3, 4, 5, 6, 7, 8, 9, 10, 11, 12, 13, or 14.
(37) The method according to any one of items (7) to (12), (35), and (36), the cell aggregate according to any one of items (13), (35), and (36), the cell culture composition according to any one of items (20), (35) and (36), or the cell culture medium according to any one of items (34) to (36), wherein a size of the widest portion in 70% or more (by weight) of the cell aggregates is 500 µm or less, preferably 300 µm or less.
(38) The method according to any one of items (7) to (12) and (35) to (37), the cell aggregate according to any one of items (13) and (35) to (37), the cell culture composition according to any one of items (20) and (35) to (37), or the cell culture medium according to any one of items (34) to (37), wherein a size of the widest portion in 70% or more (by weight) of the cell aggregates is 40 µm or more, preferably 100 µm or more.

The text of specification includes disclosure of JP Patent Application No. 2017-253147, of which the present application claims priority.

### Advantageous Effects of Invention

One embodiment of the cell aggregation suppressor of the present invention can be mixed in a culture medium to suppress aggregation of cells during suspension culture.

According to one embodiment of the method for suppressing aggregation of cells of the present invention, aggregation of cells during suspension culture can be suppressed.

According to one embodiment of the method for producing a cell aggregate of the present invention, cell aggregates can be produced in high yields.

One embodiment of the cell aggregate of the present invention has an appropriate size and high viable cell ratio.

One embodiment of the cell culture composition of the present invention can be used to produce cell aggregates in high yields.

### Brief Description of the Drawings

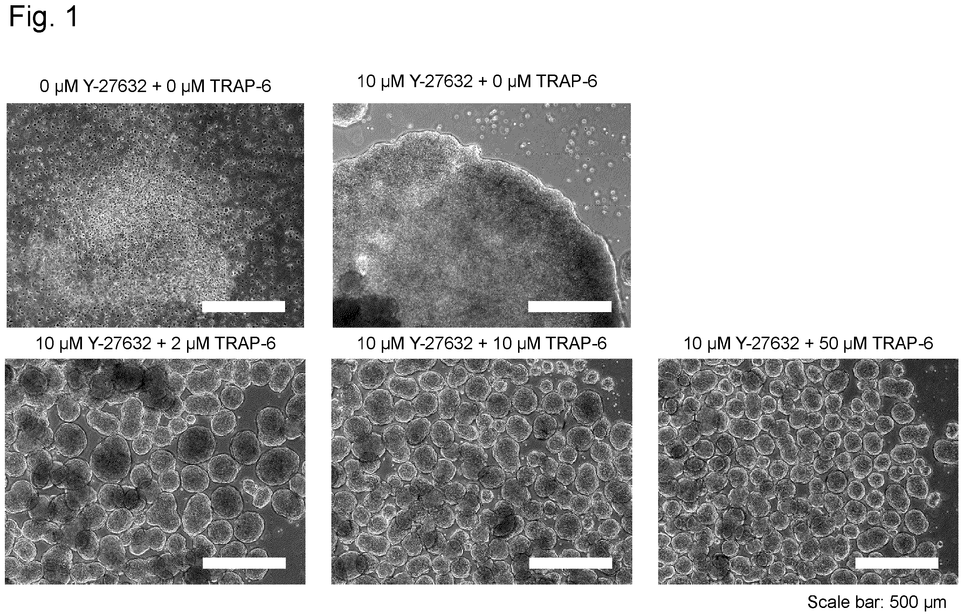

[Figure 1] Figure 1 shows observation images by phase contrast microscopy at Day 1 of culture when human iPS cells were cultured in suspension in a culture medium comprising TRAP-6 at different concentrations (0 µM, 2 µM, 10 µM, 50 µM) in the presence of Y-27632 (10 µM). The control is an observation image by phase contrast microscopy at Day 1 of culture when human iPS cells were cultured in suspension in a culture medium free of both Y-27632 and TRAP-6. The scale bar in the image data is 500 µm.

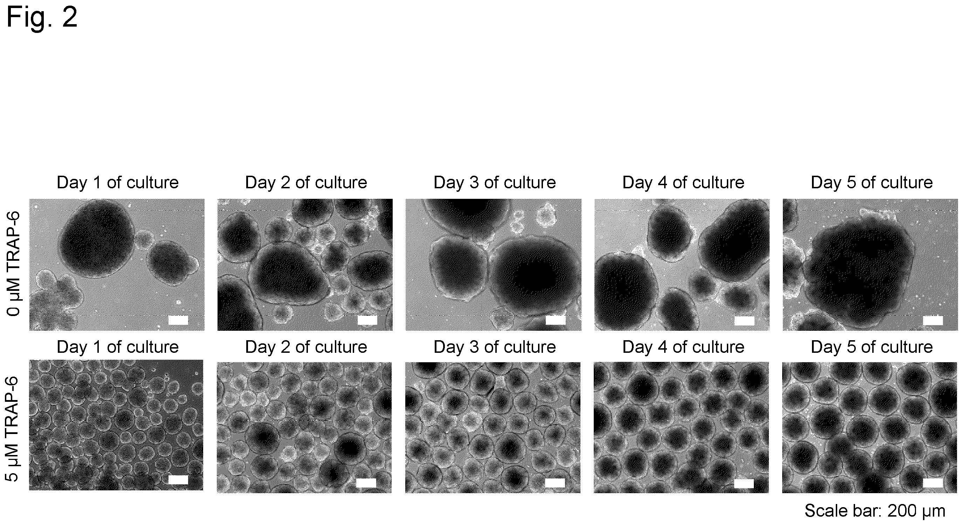

[Figure 2] Figure 2 shows observation images by phase contrast microscopy from Day 1 to Day 5 of culture when human iPS cells were cultured in suspension in a culture medium comprising 5 µM of TRAP-6 (Day 1 of culture) to produce cell aggregates and then continuously cultured in suspension in a culture medium free of TRAP-6 (Day 2 to Day 5 of culture). The controls are observation images by phase contrast microscopy from Day 1 to Day 5 of culture when human iPS cells were cultured in a culture medium free of TRAP-6.

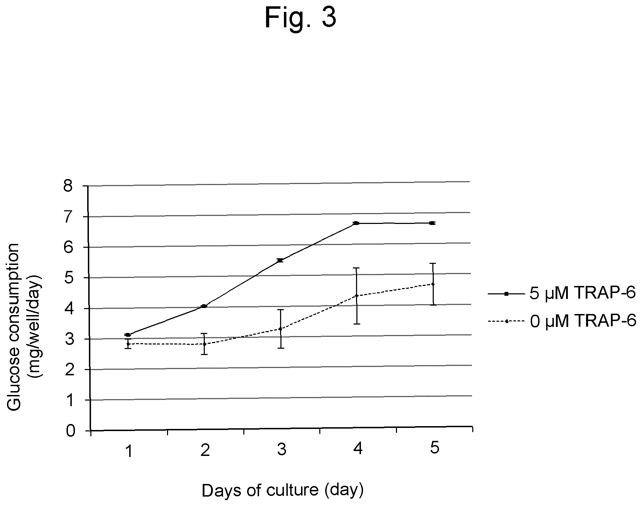

[Figure 3] Figure 3 shows the measurement result of glucose consumption from Day 1 to Day 5 of culture when human iPS cells were cultured in suspension in a culture medium comprising 5 µM of TRAP-6 (Day 1 of culture) to produce cell aggregates and then continuously cultured in suspension in a culture medium free of TRAP-6 (Day 2 to Day 5 of culture). The control is glucose consumption from Day 1 to Day 5 of culture when human iPS cells were cultured in a culture medium free of TRAP-6.

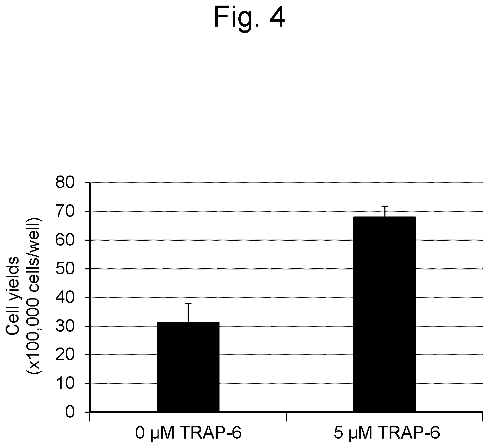

[Figure 4] Figure 4 shows the measurement result of cell yield at Day 5 of culture when human iPS cells were cultured in suspension in a culture medium comprising 5 µM of TRAP-6 (Day 1 of culture) to produce cell aggregates and then continuously cultured in suspension in a culture medium free of TRAP-6 (Day 2 to Day 5 of culture). The control is a cell yield at Day 5 of culture when human iPS cells were cultured in a culture medium free of TRAP-6.

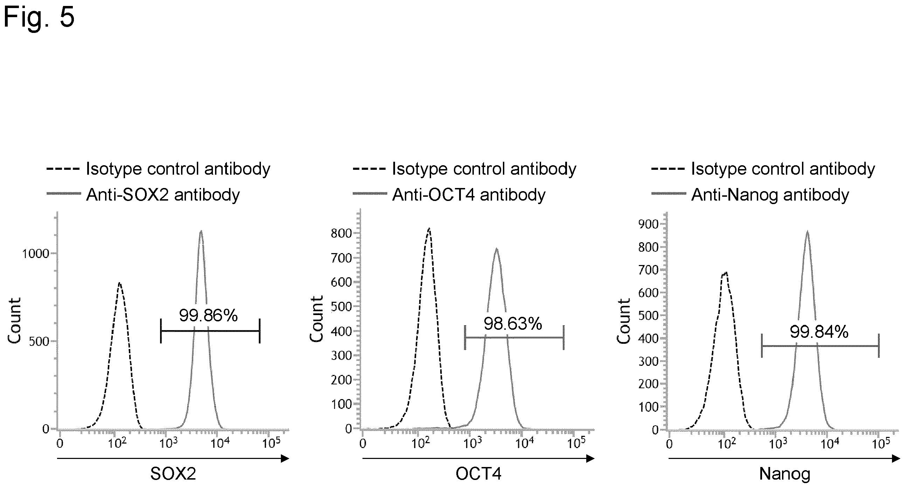

[Figure 5] Figure 5 shows the measurement results of the percentage of cells positive for undifferentiation markers (SOX2, OCT4, and Nanog) at Day 5 of culture when human iPS cells were cultured in suspension in a culture medium comprising 5 µM of TRAP-6 (Day 1 of culture) to produce cell aggregates and then continuously cultured in suspension in a culture medium free of TRAP-6 (Day 2 to Day 5 of culture).

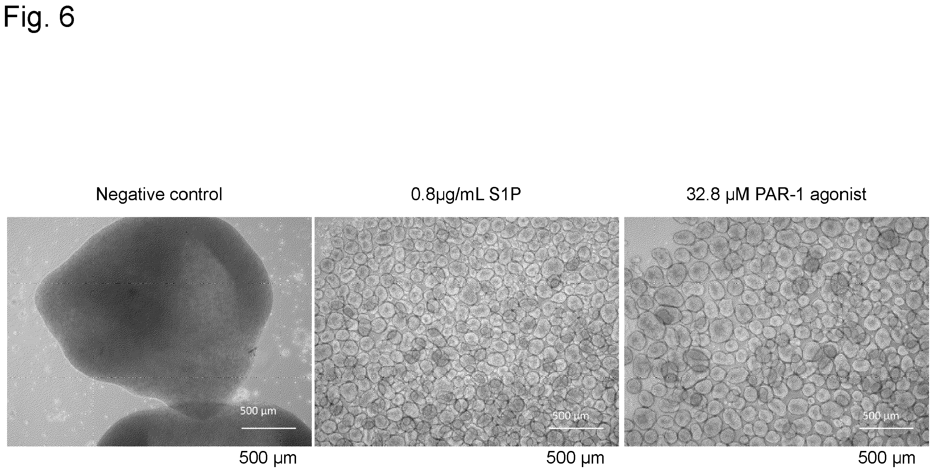

[Figure 6] Figure 6 shows an observation image by phase contrast microscopy at Day 1 of culture when human iPS cells were cultured in suspension in a culture medium comprising a PAR-1 agonist (32.8 µM) in the presence of Y-27632 (10 µM). The negative control shows an observation image by phase contrast microscopy at Day 1 of culture when human iPS cells were cultured in suspension in a culture medium comprising Y-27632 (10 µM). The positive control shows an observation image by phase contrast microscopy at Day 1 of culture when human iPS cells were cultured in suspension in a culture medium comprising S1P (0.8 µg/mL) in the presence of Y-27632 (10 µM). The scale bar in the image data is 500 µm.

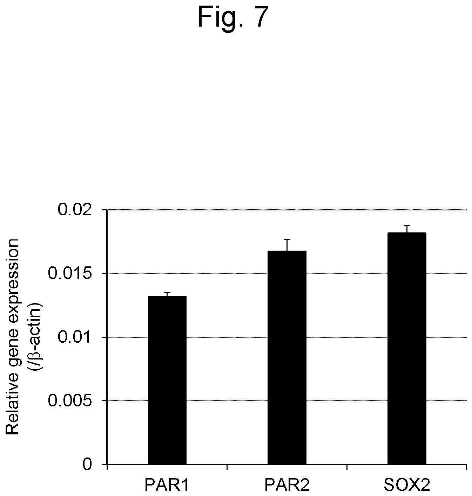

[Figure 7] Figure 7 shows the results of relative gene expression of PAR1 and PAR2 in human iPS cells (TkDN4M cell line) as measured by quantitative RT-PCR. As a positive control, SOX2, an undifferentiation marker gene, was used.

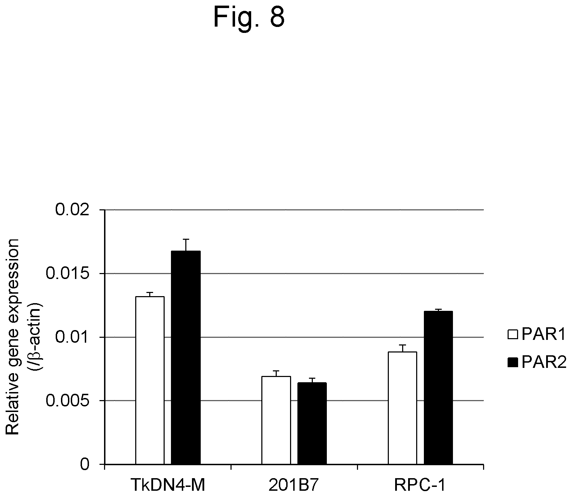

[Figure 8] Figure 8 shows the result of relative gene expression of PAR1 and PAR2 in human iPS cells (TkDN4M cell line, 201B7 cell line, RPChiPS771-2 (RPC-1 in the Figure) cell line) as measured by quantitative RT-PCR.

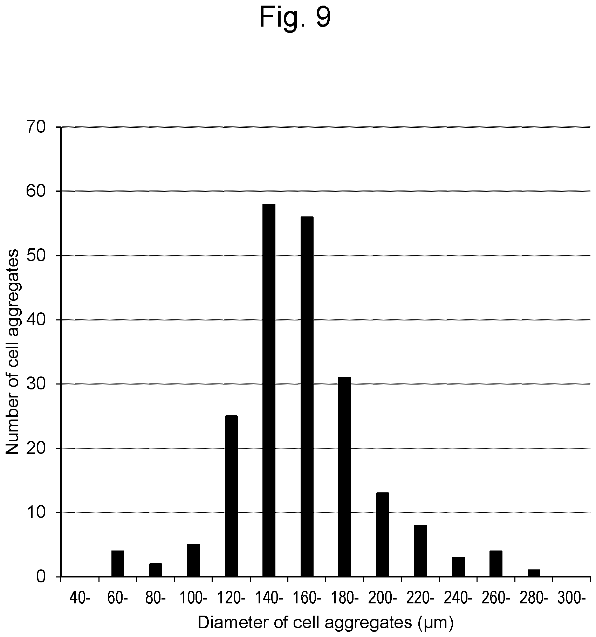

[Figure 9] Figure 9 is a graph showing the distribution of diameters of cell aggregates at Day 1 of culture when human iPS cells were cultured in suspension in a culture medium comprising 5 µM of TRAP-6 (Day 1 of culture) to produce cell aggregates.

### Description of Embodiments

Hereinafter, preferable embodiments of the present invention will be described in detail.

### <1. Cells>

Aggregate-forming cells in the present invention may be cells that are adherent (adherent cells). Examples of the adherent cells may include: animal-derived cells; preferably mammalian-derived cells; more preferably biological tissue-derived cells and cells derived from the biological tissue-derived cells; particularly preferably epithelial tissue-derived cells and cells derived from the epithelial tissue-derived cells, connective tissue-derived cells and cells derived from the connective tissue-derived cells, muscular tissue-derived cells and cells derived from the muscular tissue-derived cells, or nervous tissue-derived cells and cells derived from the nervous tissue-derived cells; further more preferably animal-derived stem cells and cells differentiated from the animal-derived stem cells; still more preferably animal-derived pluripotent stem cells and cells differentiated from the animal-derived pluripotent stem cells; still more preferably mammalian-derived pluripotent stem cells and cells differentiated from the mammalian-derived pluripotent stem cells; and most preferably human-derived pluripotent stem cells and cells differentiated from the human-derived pluripotent stem cells.

As used herein, the "stem cell" is a cell that is capable of differentiation into another cell and has a self-replicating activity. Among the "stem cells", a cell that has a multipotency (pluripotency) capable of differentiating into all types of cells constituting a living body and that can continue to proliferate infinitely while maintaining its pluripotency during *in vitro* culture under suitable conditions is referred to as a "pluripotent stem cell". Specific examples of the pluripotent stem cells include, but are not limited to, embryonic stem cells (ES cells), EG cells, which are pluripotent stem cells derived from fetal primordial germ cells, (Shamblott M. J. et al., Proc. Natl. Acad. Sci. USA. (1998) 95, p.13726-13731), GS cells, which are testis-derived pluripotent stem cells, (Conrad S., Nature (2008) 456, p.344-349), and iPS cells (induced pluripotent stem cells), which are somatic cell-derived induced pluripotent stem cells.

Regarding the pluripotent stem cells used in the present invention, particularly preferred are ES cells or iPS cells. ES cells are pluripotent stem cells derived from an early embryo. iPS cells are cultured cells produced by introducing reprogramming factors into a somatic cell, so that the somatic cell is reprogrammed into an undifferentiated state and is given pluripotency. Examples of the reprogramming factors that can be used include OCT3/4, KLF4, SOX2, and c-Myc (Takahashi K, et al. Cell. 2007; 131:861-72). For example, OCT3/4, SOX2, LIN28, and Nanog may be used (Yu J, et al. Science. 2007; 318:1917-20). Examples of how to introduce these factors into a cell include, but are not particularly limited to, a plasmid-mediated gene transfer, synthetic RNA introduction, and a direct injection of a protein(s). In addition, it may be possible to use iPS cells that are created using, for example, microRNA, RNA, and/or a low-molecular-weight compound. As the pluripotent stem cells (including the ES cells, iPS cells, etc.), commercially available products or cells obtained from a third party may be used or freshly prepared ones may be used. Examples of iPS cell lines that can be used include 253G1, 201B6, 201B7, 409B2, 454E2, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC 38-2, MSC-iPSC1, and BJ-iPSC1. Examples of ES cell lines that can be used include KhES-1, KhES-2, KhES-3, KhES-4, KhES-5, SEES-1, SEES-2, SEES-3, SEES-4, SEES-5, SEES-6, SEES-7, HUES8, CyT49, H1, H9, HS-181, and RPChiPS771-2. Also, freshly prepared clinical-grade iPS or ES cells may be used. Examples of the origin of cells when iPS cells are created include, but are not particularly limited to, fibroblasts and lymphocytes.

The types of cells in the present invention are not particularly limited as long as they are cells capable of adhering to plastics or cells by extracellular matrices, cadherins, or the like. Examples thereof include pluripotent stem cells described above (e.g., induced pluripotent stem cells (iPS cells), embryonic stem cells (ES cells), GS cells that are pluripotent stem cells derived from testis, EG cells derived from fetal primordial germ cells, or Muse cells derived from bone marrow or the like), somatic stem cells (e.g., mesenchymal stem cells derived from bone marrow, adipose tissue, dental marrow, placenta, ovum, umbilical cord blood, amniotic membrane, chorionic membrane or the like, or neural stem cells), neuronal cells, cardiomyocytes, cardiomyocardial progenitor cells, hepatocytes, hepatic progenitor cells, α cells, β cells, fibroblasts, chondrocytes, corneal cells, vascular endothelial cells, vascular endothelial progenitor cells, and peripheral cells. The cells may be in a form into which a gene is introduced or a form in which a gene of interest on the genome is knockdowned.

Cells used in the present invention may be originated from any animal. Examples of the origin may include: mammals such as rodents (e.g., a mouse, rat, hamster), primates (e.g., a human, gorilla, chimpanzee), and domestic animals and pets (e.g., a dog, cat, rabbit, cow, horse, sheep, goat). Particularly preferred are human cells.

In the present invention, cells isolated after undergoing adherent or suspension culture may be used. Here, the term "isolated cells" means cells obtained by detaching and dispersing a cell population composed of a plurality of cells adhering to one another. The isolation involves the step of detaching and dispersing cells adhering to, for example, cultureware and/or a culture support or a cell population, in which cells adhere to one another, to give single cells. The cell population to be isolated may be in suspension in a liquid culture medium. Preferable examples of the isolation procedure may include, but are not particularly limited to, a procedure using a detachment agent (e.g., a cell detachment enzyme such as trypsin or collagenase), a chelating agent (e.g., EDTA (ethylene diamine tetraacetic acid)), or a mixture of the detachment agent and the chelating agent. Examples of the detachment agent include, but are not particularly limited to, trypsin, Accutase (a registered trade mark), TrypLE™ Express Enzyme (Life Technologies Japan Ltd.), TrypLE™ Select Enzyme (Life Technologies Japan Ltd.), "Dispase" (a registered trade mark), and collagenase. The cells that have been isolated, frozen, and stored after the isolation procedure may be preferably used in the present invention.

### <2. Cell Aggregates>

A cell aggregate refers to what is called a spheroid that is a clustered cell population formed while a plurality of cells aggregate three-dimensionally. The cell aggregate typically has a generally spherical shape.

In the present invention, cells that constitute a cell aggregate are not particularly limited as long as they are one or more type of cells described above. For example, a cell aggregate composed of pluripotent stem cells such as human pluripotent stem cells or human embryonic stem cells includes cells expressing a pluripotent stem cell marker and/or positive for a pluripotent stem cell marker. Examples of the pluripotent stem cell maker include alkaline phosphatase, NANOG, OCT4, SOX2, TRA-1-60, c-Myc, KLF4, LIN28, SSEA-4, and SSEA-1.

The pluripotent stem cell marker can be detected by any detection method in the art. Examples of the method for detecting expression markers include, but are not limited to, flow cytometry. In flow cytometry using a fluorescently labeled antibody, when cells emitting greater fluorescence compared to negative control (isotype control) are detected, the cells are determined to be "positive" for the marker. The percentage of cells positive for fluorescently labeled antibodies analyzed by flow cytometry is sometimes referred to as a positive ratio. As the fluorescently labeled antibodies, any antibody known in the art can be used, and examples of the antibodies include, but are not limited to, antibodies labeled with fluorescein isothiocyanate (FITC), phycoerythrin (PE), allophycocyanin (APC), or the like.

When cells that constitute the cell aggregate are pluripotent stem cells, the percentage (ratio) of cells that express pluripotent stem cell markers and/or are positive for pluripotent stem cell markers may be, for example, 80% or more, 90% or more, 91% or more, 92% or more, 93% or more, 94% or more, 95% or more, 96% or more, 97% or more, 98% or more, 99% or more, or 100% or less. The cell aggregates in which the percentage of cells that express pluripotent stem cell markers and/or are positive for pluripotent stem cell markers is within the above-mentioned range are a more undifferentiated and more homogeneous cell population. Note that pluripotent stem cell markers are synonymous with undifferentiation markers, and both can be used interchangeably.

The size of the cell aggregate produced by one or more embodiments of the present invention is not particularly limited, and when observed under a microscope, the upper limit of the size of the widest portion in an observation image is, for example, 1000 µm or less, 900 µm or less, 800 µm or less, 700 µm or less, 600 µm or less, 500 µm or less, 400 µm or less, 300 µm or less, or 200 µm or less. The lower limit is, for example, 50 µm or more, 60 µm or more, 70 µm or more, 80 µm or more, 90 µm or more, or 100 µm or more. The cell aggregates with such a size range have a preferable cell proliferation environment because oxygen and nutritional components are easily supplied to their inner cells.

In a cell aggregate population produced by one or more embodiments of the present invention, for example, 10% or more, 20% or more, 30% or more, 40% or more, 50% or more, 60% or more, 70% or more, 80% or more, or 90% or more by weight of the cell aggregates constituting the cell aggregate population can have a size within the above-mentioned range. The cell aggregate population including 20% or more of cell aggregates having a size within the above-mentioned range has a preferable cell proliferation environment because oxygen and nutritional components are easily supplied to their inner cells in individual cell aggregates.

It is preferable in a cell aggregate population produced by one or more embodiments of the present invention that the percentage of viable cells (viability) in cells constituting the cell aggregate population is, for example, 50% or more, 60% or more, 70% or more, 80% or more, or 90% or more. The cell aggregates having a viability within the above-mentioned range easily maintain the aggregate state and are in a preferred state for cell proliferation.

### <3. Culture Medium>

The culture medium used in the present invention can be prepared by using any culture medium for culturing an animal cell as a basal medium, and appropriately adding an agonist of a thrombin receptor or a cell aggregation suppressor comprising an agonist of a thrombin receptor, and other components as needed, to the basal medium. It is preferable that the culture medium used in the present invention is suitable for suspension culture of cells, and typically is a liquid culture medium.

Examples of the basal medium that can be used include, but are not particularly limited to, BME medium, BGJb medium, CMRL1066 medium, Glasgow MEM medium, Improved MEM Zinc Option medium, IMDM medium (Iscove's Modified Dulbecco's Medium), Medium 199 medium, Eagle MEM medium, αMEM medium, DMEM medium (Dulbecco's Modified Eagle's Medium), Ham's F10 medium, Ham's F12 medium, RPMI 1640 medium, Fischer's medium, and a mixed medium thereof (e.g., DMEM/F12 medium (Dulbecco's Modified Eagle's Medium/Nutrient Mixture F-12 Ham)). The DMEM/F12 medium may be used, in particular, by mixing DMEM medium and Ham's F12 medium in a weight ratio of, for example, from 60/40 or more to 40/60 or less, from 55/45 or more to 45/55 or less, or in a weight ratio of 50/50.

The culture medium used in the present invention is preferably a medium containing no serum, namely a serum-free medium. The culture medium used in the present invention preferably contains at least one selected from L-ascorbic acid, insulin, transferrin, selenium and sodium bicarbonate, and more preferably contains all of these. The L-ascorbic acid, insulin, transferrin, selenium, and sodium bicarbonate may be added to the medium in the form of, for example, a solution, derivative, salt, or mixed reagent. For example, L-ascorbic acid may be added to the medium in the form of a derivative such as magnesium-ascorbyl-2-phosphate. Selenium may be added to the medium in the form of a selenite (e.g., sodium selenite). The insulin and transferrin may be natural ones isolated from a tissue or serum of an animal (e.g., preferably a human, mouse, rat, cow, horse, goat). They may be genetically engineered recombinant proteins. The insulin, transferrin, and selenium may be added to the medium in the form of a reagent ITS (insulin-transferrin-selenium). The ITS is a cell growth-promoting additive containing insulin, transferrin, and sodium selenite.

A commercially available culture medium containing at least one selected from L-ascorbic acid, insulin, transferrin, selenium, and sodium bicarbonate may be used. Examples of a commercially available culture medium supplemented with insulin and transferrin may include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE™ (BioWhittaker, Inc.), UltraDOMA™ (BioWhittaker, Inc.), UltraCHO™ (BioWhittaker, Inc.), and UltraMDCK™ (BioWhittaker, Inc.). For, example, STEMPRO (a registered trade mark), hESC SFM (Life Technologies Japan Ltd.), mTeSR1 (Veritas, Ltd.), or TeSR2 (Veritas, Ltd.) may be preferably used. In addition, it is preferable to use a culture medium used for culturing human iPS cells and/or human ES cells.

The culture medium used in the present invention preferably contains at least one growth factor. The liquid culture medium preferably contains at least one growth factor, which is not limited to the following, selected from the group consisting of FGF2 (basic fibroblast growth factor-2), TGF-β1 (transforming growth factor-β1), Activin A, IGF-1, MCP-1, IL-6, PAI, PEDF, IGFBP-2, LIF, and IGFBP-7. Particularly preferred growth factor is FGF2 and/or TGF-β1.

The most preferable culture medium used in the present invention is a serum-free medium containing, in addition to the agonist of thrombin receptor described below, components: L-ascorbic acid, insulin, transferrin, selenium, and sodium bicarbonate as well as at least one growth factor. Particularly preferred is a serum-free DMEM/F12 medium containing L-ascorbic acid, insulin, transferrin, selenium, and sodium bicarbonate as well as at least one growth factor (preferably, FGF2 and TGF-β1). Examples of such a culture medium that can be preferably used include Essential 8™ culture medium (Life Technologies Japan Ltd.) supplemented with an agonist of thrombin receptor. The Essential 8™ medium may be prepared by mixing DMEM/F-12 (HAM) (1:1), which is a DMEM/F12 medium marketed by Life Technologies Japan Ltd., and Essential 8™ supplement (containing L-ascorbic acid, insulin, transferrin, selenium, sodium bicarbonate, FGF2, and TGF-β1).

The culture medium used in the present invention may contain further components such as fatty acids or lipids, amino acids (e.g., non-essential amino acids), vitamins, cytokines, antioxidants, 2-mercaptoethanol, pyruvic acid, buffers, inorganic salts, antibiotics and kinase inhibitors.

Examples of the antibiotics that can be used include penicillin, streptomycin, and amphotericin B.

Examples of the kinase inhibitors that can be added include ROCK inhibitors. The ROCK inhibitors are defined as a substance that inhibits the kinase activity of Rho kinase (ROCK, Rho-associated protein kinase). Examples thereof include Y-27632 (4-[(1R)-1-aminoethyl]-N-pyridin-4-ylcyclohexane-1-carboxamide or a salt thereof (e.g., dihydrochloride)) (see, e.g., Ishizaki et al., Mol. Pharmacol. 57, 976-983 (2000); Narumiya et al., Methods Enzymol. 325, 273-284 (2000)), H-1152 ((S)-(+)-2-methyl-1-[(4-methyl-5-isoquinolinyl)sulfonyl]-hexahydro-1H-1,4-diazepine or a salt thereof (e.g., dihydrochloride)) (see, e.g., Sasaki et al., Pharmacol. Ther. 93: 225-232 (2002)), Fasudil/HA1077 (1-(5-isoquinolinsulfonyl)homopiperazine or a salt thereof (e.g., dihydrochloride)) (see, e.g., Uenata et al., Nature 389: 990-994 (1997)), Wf-536 ((+)-(R)-4-(1-aminoethyl)-N-(4-pyridyl)benzamide monohydrochloride) (see, e.g., Nakajima et al., CancerChemother. Pharmacol. 52(4): 319-324 (2003)), and derivatives thereof, as well as antisense nucleic acids, RNA interference-induced nucleic acids (e.g., siRNA), dominant negative variants to ROCK, and expression vectors thereof. In addition, other low-molecular-weight compounds have also been known as the ROCK inhibitors, and such compounds or derivatives thereof may be used as the ROCK inhibitors in accordance with the present invention (see, for example, US Patent Application Publication Nos. 2005/0209261, 2005/0192304, 2004/0014755, 2004/0002508, 2004/0002507, 2003/0125344, and 2003/0087919, and WO2003/062227, WO2003/059913, WO2003/062225, WO2002/076976, and WO2004/039796). As the ROCK inhibitor, one or two or more ROCK inhibitors can be used.

The structural formula of 4-[(1R)-1-aminoethyl]-N-pyridin-4-ylcyclohexane-1-carboxamide described above is as follows:

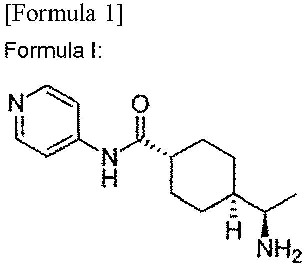

The ROCK inhibitor is particularly preferably one or more selected from Y-27632 and H-1152, and most preferably Y-27632. Y-27632 and H-1152 may be used in the form of hydrates, respectively.

The concentration of the ROCK inhibitor such as Y-27632 in a liquid culture medium is not particularly limited, and particularly preferably in the range of, for example, 3.3 ng/mL or more and 3.4 mg/mL or less. The lower limit of the concentration is not particularly limited as long as the effect is exhibited at the concentration, and for example, 33 ng/mL or more, 330 ng/mL or more, or 800 ng/mL or more, for example, 1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, 10 µg/mL or more, 11 µg/mL or more, 12 µg/mL or more, or 13 µg/mL or more. The upper limit of the concentration is not particularly limited as long as the cells are not to be killed at the concentration, and for example, 340 µg/mL or less, 300 µg/mL or less, 200 µg/mL or less, 100 µg/mL or less, 90 µg/mL or less, 80 µg/mL or less, 70 µg/mL or less, 60 µg/mL or less, 50 µg/mL or less, 40 µg/mL or less, 34 µg/mL or less, 30 µg/mL or less, 25 µg/mL or less, 20 µg/mL or less, 19 µg/mL or less, 18 µg/mL or less, 17 µg/mL or less, 16 µg/mL or less, 15 µg/mL or less, or 14 µg/mL or less. It is also particularly preferable that the concentration of the ROCK inhibitor in the culture medium is in the range of 10 nM or more and 10 mM or less. The concentration is, for example, 100 nM or more, 1 µM or more, or 2.5 µM or more, and may be, for example, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, 20 µM or more, 21 µM or more, 22 µM or more, 23 µM or more, 24 µM or more, 25 µM or more, 26 µM or more, 27 µM or more, 28 µM or more, 29 µM or more, 30 µM or more, 31 µM or more, 32 µM or more, 33 µM or more, 34 µM or more, 35 µM or more, 36 µM or more, 37 µM or more, 38 µM or more, 39 µM or more, or 40 µM or more. The upper limit is, for example, 1 mM or less, 900 µM or less, 800 µM or less, 700 µM or less, 600 µM or less, 500 µM or less, 400 µM or less, 300 µM or less, 200 µM or less, 100 µM or less, 90 µM or less, 80 µM or less, 70 µM or less, 60 µM or less, 50 µM or less, or 40 µM or less.

### <4. Agonists of Thrombin Receptor>

As used herein, the "thrombin receptor" refers to a receptor activated by thrombin, i.e., PAR-1 (protease-activated receptor-1), PAR-3 (protease-activated receptor-3), or PAR-4 (protease-activated receptor-4), further preferably PAR-1 or PAR-4, most preferably PAR-1. PAR-2, which is known as a PAR family member, is not a receptor of thrombin, but is a transmembrane receptor that is activated by trypsin, tryptase, blood coagulation factors, and the like, and when PAR-2 is activated, signals are transmitted into cells similarly to other PAR family members (PAR-1, PAR-3, PAR-4). Thus, those skilled in the art would readily expect that PAR-2 agonists would achieve the same effect as the agonists of a thrombin receptor. Thus, the "thrombin receptor" as used herein also includes PAR-2 in addition to PAR-1, PAR-3 and PAR-4 described above. As used herein, the "agonist of a thrombin receptor" is defined herein as a substance that activates a thrombin receptor and includes both endogenous and exogenous agonists. Examples of the agonist of a thrombin receptor include, but are not limited to, serine proteases such as thrombin (PAR-1, PAR-3, and PAR-4), trypsin, blood coagulation factor Xa, plasmin (PAR-1, PAR-2, and PAR-4), activated protein C (PAR-1), tryptase and matriptase (PAR-2), and cathepsin G (PAR-4) (see, e.g., Tejminder S. Sidhu et al., Int. J. Mol. Sci. 2014, 15, 6169-6183). In addition to these serine proteases, peptides comprising the amino acid sequence that occurs after the thrombin receptor undergoes cleavage by the serine protease are also included in the agonist of a thrombin receptor. Such peptides can be readily identified by those skilled in the art based on the amino acid sequence of the thrombin receptor in an animal species of interest and the site of cleavage by the protease. For example, peptides comprising the amino acid sequences SFLLR (SEQ ID NO: 1) and SFFLR (SEQ ID NO: 2) can be used as agonists of human PAR-1 and mouse PAR-1, respectively; peptides comprising the amino acid sequences TFRGAP (SEQ ID NO: 3) and SFGBGGP (SEQ ID NO: 4) can be used as agonists of human PAR-3 and mouse PAR-3, respectively; and peptides comprising the amino acid sequences GYPGQV (SEQ ID NO: 5) and GYPGFK (SEQ ID NO: 6) can be used as agonists of human PAR-4 and mouse PAR-4, respectively (see, e.g., Tomiko Sekiguchi, YAKUGAKU ZASSHI (Journal of the Pharmaceutical Society of Japan), 2005, 125(6), 491-498). A peptide comprising the amino acid sequence SFLLRN (SEQ ID NO: 7) and a peptide comprising the amino acid sequence TFLLRN (SEQ ID NO: 8) can be used as an agonist of PAR-1. Examples of particularly preferred agonists include a peptide consisting of the amino acid sequence SFLLRN (SEQ ID NO: 7), i.e., TRAP-6 (Thrombin Receptor Activator for Peptide 6), and a peptide consisting of the amino acid sequence TFLLRN (SEQ ID NO: 8), which act as PAR-1 agonists.

Peptides comprising the amino acid sequence TFLLRNPNDK (SEQ ID NO: 9), the amino acid sequence FSLLRN (SEQ ID NO: 10), and the amino acid sequence FLLRN (SEQ ID NO: 11) can be used as agonists of PAR-1. Peptides comprising the amino acid sequence SLIGKV (SEQ ID NO: 12) and the amino acid sequence SLIGRL (SEQ ID NO: 13) can be used as agonists of PAR-2. A peptide comprising the amino acid sequence AYPGKF (SEQ ID NO: 14) can be used as an agonist of PAR-4.

Further examples of the agonist of a thrombin receptor include a peptide comprising an amino acid sequence derived from the amino acid sequence of SEQ ID NO: 1 to 8 by substitution, deletion, insertion, and/or addition of one or plurality of amino acids. The "plurality of amino acids" is not particularly limited in number of amino acids as long as the peptide can activate a thrombin receptor like peptides comprising the amino acid sequence of SEQ ID NO: 1 to 8.

Further examples of the agonist of a thrombin receptor include a peptide comprising an amino acid sequence derived from the amino acid sequence of SEQ ID NO: 1 to 8 by substitution, deletion, insertion, and/or addition of one or two amino acids. An agonist of a thrombin receptor may be a peptide consisting of any one of the above amino acid sequences.

Derivatives such as C-terminal amidated products of the above peptides are also included in the agonist of a thrombin receptor. Further examples of the agonist of a thrombin receptor include agonist antibodies, expression vectors of the above-mentioned peptides or agonist antibodies, and low-molecular-weight compounds. As the agonist of a thrombin receptor, one or two or more agonists of a thrombin receptor can be used.

The sequences of SEQ ID NOs: 1 to 14 have the backbone X-Phe-X-Leu-Arg, as an commonly conserved amino acid sequence. This backbone enables the peptides to function as an agonist of thrombin. Note that any amino acid can be appropriately selected for X.

Further examples of the agonist of a thrombin receptor include a peptide comprising an amino acid sequence derived from the amino acid sequence of SEQ ID NO: 9 to 14 by substitution, deletion, insertion, and/or addition of one or plurality of amino acids. The "plurality of amino acids" is not particularly limited in number of amino acids as long as the peptide can activate a thrombin receptor like peptides comprising the amino acid sequence of SEQ ID NO: 1 to 8.

Further examples of the agonist of a thrombin receptor include a peptide comprising an amino acid sequence derived from the amino acid sequence of SEQ ID NO: 9 to 14 by substitution, deletion, insertion, and/or addition of one or two amino acids. The agonist of a thrombin receptor may be a peptide consisting of any one of the above amino acid sequences.

The agonist of a thrombin receptor may be a peptide having 60% or more sequence identity to any of the above amino acid sequences (peptides consisting of the amino acid sequence of SEQ ID NO: 1 to 14) and being capable of activating the thrombin receptor. The sequence identity may be 66% or more, 75% or more, 80% or more, 83% or more, 85% or more, 90% or more, 91% or more, 92% or more, 93% or more, 94% or more, 95% or more, 96% or more, 97% or more, 98% or more, or 99% or more.

Examples of the agonist of a thrombin receptor include, in addition to peptides consisting of the amino acid sequence of SEQ ID NO: 7 or SEQ ID NO: 8, peptides consisting of an amino acid sequence having 60% or more sequence identity to the amino acid sequence of SEQ ID NO: 7 or SEQ ID NO: 8. The sequence identity may be 80% or more, 83% or more, 85%, 90% or more, 91% or more, 92% or more, 93% or more, 94% or more, 95% or more, 96% or more, 97% or more, 98% or more, 99% or more, or 100%.

Sequence identity of amino acids can be determined using methods well known to those skilled in the art, sequence analysis software, and the like. Examples thereof include a blastp program of BLAST algorithm and a fasta program of FASTA algorithm. The sequence identity of amino acid sequences here is a value obtained by comparing the amino acid sequence to be evaluated with the amino acid sequence, for example, of SEQ ID NO: 7 or 8, and expressing the frequency with which identical amino acids occur at the same site by %.

For example, when a sequence identity between SEQ ID NO: 8 and SEQ ID NO: 2 is analyzed using a sequence analysis software "GENETYX Network™ Ver. 13.0.2" (GENETYX CORPORATION), the sequence identity is calculated as 60%.

For example, when a sequence identity between SEQ ID NO: 8 and SEQ ID NO: 1 is analyzed using a sequence analysis software "GENETYX Network™ Ver. 13.0.2" (GENETYX CORPORATION), the sequence identity is calculated as 80%.

For example, when a sequence identity between SEQ ID NO: 7 and SEQ ID NO: 8 is analyzed using a sequence analysis software "GENETYX Network™ Ver. 13.0.2" (GENETYX CORPORATION), the sequence identity is calculated as 83%.

For example, when a sequence identity between SEQ ID NO: 7 and SEQ ID NO: 1 is analyzed using a sequence analysis software "GENETYX Network™ Ver. 13.0.2" (GENETYX CORPORATION), the sequence identity is calculated as 100%.

Examples of the agonist of a thrombin receptor include, in addition to peptides consisting of the amino acid sequence of SEQ ID NO: 12 or SEQ ID NO: 13, peptides consisting of amino acid sequences having 66% or more sequence identity to the amino acid sequence of SEQ ID NO: 12 or SEQ ID NO: 13. The sequence identity may be 75% or more, 80% or more, 83% or more, 85%, 90% or more, 91% or more, 92% or more, 93% or more, 94% or more, 95% or more, 96% or more, 97% or more, 98% or more, 99% or more, or 100%.

Sequence identity of amino acids can be determined using methods well known to those skilled in the art, sequence analysis software, and the like. Examples thereof include a blastp program of BLAST algorithm and a fasta program of FASTA algorithm. The sequence identity of amino acid sequences here is a value obtained by comparing the amino acid sequence to be evaluated with the amino acid sequence, for example, of SEQ ID NO: 12 or 13, and expressing the frequency with which identical amino acids occur at the same site by %.

For example, when a sequence identity between SEQ ID NO: 12 and SEQ ID NO: 13 is analyzed using a sequence analysis software "GENETYX Network™ Ver. 13.0.2" (GENETYX CORPORATION), the sequence identity is calculated as 66%.

Examples of the agonist of a thrombin receptor include, in addition to peptides consisting of the amino acid sequence of SEQ ID NO: 5, SEQ ID NO: 6, or SEQ ID NO: 14, peptides consisting of amino acid sequences having 60% or more sequence identity to the amino acid sequence of SEQ ID NO: 5 or SEQ ID NO: 6. The sequence identity may be 75% or more, 80% or more, 83% or more, 85%, 90% or more, 91% or more, 92% or more, 93% or more, 94% or more, 95% or more, 96% or more, 97% or more, 98% or more, 99% or more, or 100%.

Sequence identity of amino acids can be determined using methods well known to those skilled in the art, sequence analysis software, and the like. Examples thereof include a blastp program of BLAST algorithm and a fasta program of FASTA algorithm. The sequence identity of amino acid sequences here is a value obtained by comparing the amino acid sequence to be evaluated with the amino acid sequence, for example, of SEQ ID NO: 5 or 6, and expressing the frequency with which identical amino acids occur at the same site by %.

For example, when a sequence identity between SEQ ID NO: 5 and SEQ ID NO: 14 is analyzed using a sequence analysis software "GENETYX Network™ Ver. 13.0.2" (GENETYX CORPORATION), the sequence identity is calculated as 60%.

For example, when a sequence identity between SEQ ID NO: 6 and SEQ ID NO: 14 is analyzed using a sequence analysis software "GENETYX Network™ Ver. 13.0.2" (GENETYX CORPORATION), the sequence identity is calculated as 75%.

For example, when a sequence identity between SEQ ID NO: 5 and SEQ ID NO: 6 is analyzed using a sequence analysis software "GENETYX Network™ Ver. 13.0.2" (GENETYX CORPORATION), the sequence identity is calculated as 100%.

### <5. Methods for Suppressing Aggregation of Cells>

One aspect of the present invention is a method for suppressing aggregation of cells, comprising a step of culturing the cells in suspension in a culture medium comprising an agonist of a thrombin receptor (suspension culture step). The method of the present invention may comprise a collection step as an optional step.

As used herein, "suppressing aggregation of cells" or "cell aggregation suppression" refers to suppressing aggregation of cells, thereby suppressing the formation or expansion of cell aggregates. As used herein, the "cell aggregation suppressor" refers to an agent that has an effect of suppressing cell aggregation.

The method of this aspect comprises the suspension culture step as an essential step, and a maintenance culture step and a collection step as optional steps. Hereinafter, each step is described below.

### (Suspension Culture Step)

Specific embodiments of the step of culturing cells in suspension in a culture medium comprising an agonist of a thrombin receptor (suspension culture step) are described.

Specific embodiments of the agonist of a thrombin receptor, culture medium, and cell are as described above.

The concentration of the agonist of a thrombin receptor in the culture medium in the suspension culture step can be adjusted appropriately according to various conditions such as type of cells, the number of cells, and type of culture medium so that aggregation of cells can be suppressed. It is particularly preferable that the concentration of the agonist of a thrombin receptor in the culture medium in the suspension culture step is in the range of 3.7 ng/mL or more and 3.8 mg/mL or less. The lower limit of the concentration is not particularly limited as long as the effect is exhibited at the concentration, and for example, 37 ng/mL or more, 40 ng/mL or more, 50 ng/mL or more, 60 ng/mL or more, 70 ng/mL or more, 80 ng/mL or more, 90 ng/mL or more, 100 ng/mL or more, 150 ng/mL or more, 200 ng/mL or more, 250 ng/mL or more, 300 ng/mL or more, 350 ng/mL or more, 370 ng/mL or more, 400 ng/mL or more, 500 ng/mL or more, 600 ng/mL or more, 700 ng/mL or more, 800 ng/mL or more, 900 ng/mL or more, or 925 ng/mL or more. The upper limit is not particularly limited as long as the cells are not to be killed at the concentration, and is 3 mg/mL or less, 2 mg/mL or less, 1 mg/mL or less, 900 µg/mL or less, 800 µg/mL or less, 700 µg/mL or less, 600 µg/mL or less, 500 µg/mL or less, 400 µg/mL or less, 380 µg/mL or less, 350 µg/mL or less, 300 µg/mL or less, 250 µg/mL or less, 200 µg/mL or less, 150 µg/mL or less, 100 µg/mL or less, 50 µg/mL or less, 38 µg/mL or less, 35 µg/mL or less, 30 µg/mL or less, 25 µg/mL or less, 20 µg/mL or less, or 15.2 µg/mL or less. It is also particularly preferable that the concentration of the agonist of a thrombin receptor in the culture medium in the suspension culture step is in the range of 5 nM or more and 5 mM or less. The concentration is, for example, 50 nM or more, 100 nM or more, 200 nM or more, 300 nM or more, 400 nM or more, 500 nM or more, 600 nM or more, 700 nM or more, 800 nM or more, 900 nM or more, 1 µM or more, 1.25 µM or more, 2 µM or more, 3 µM or more, 4 µM or more, 5 µM or more, 6 µM or more, 7 µM or more, 8 µM or more, 9 µM or more, or 10 µM or more, and 500 µM or less, 400 µM or less, 300 µM or less, 200 µM or less, 100 µM or less, 50 µM or less, 40 µM or less, 30 µM or less, or 20 µM or less. When the concentration of the agonist of a thrombin receptor in the culture medium is within the above-mentioned range, in particular, influence on undifferentiation and viability of pluripotent stem cells or the like is small and suppression on aggregation of cells is effected and the size of cell aggregates can be regulated.

The suspension culture step is preferably a step of culturing a cell in suspension under conditions in which, assuming that the agonist of a thrombin receptor of the present invention is not present in the culture medium, aggregation of cells becomes excessive. Examples of the conditions under which aggregation of cells becomes excessive include, but are not limited to, conditions under which aggregation of cells causes inadequate nutrient and/or oxygen supply to some cells and leads to cell killing and/or decreased proliferation.

The cultureware used in the suspension culture step is preferably a container on which cells adhere less to an inner surface thereof. Examples of such a container include plates, the surface of which is subjected to hydrophilic treatment with a biocompatible substance. Examples of the cultureware that may be used include, but are not particularly limited to, Nunclon™ Sphera (Thermo Fisher Scientific Inc.).

Examples of the shape of the cultureware include, but are not particularly limited to, a dish, flask, well, bag, and spinner flask shape.

The suspension culture may be static culture or may be culture under conditions in which the culture medium flows (fluid culture), but preferably fluid culture. It is preferable that the fluid culture is a culture under conditions in which the culture medium flows so as to suppress cell aggregation. Examples of the culture under conditions in which the culture medium flows so as to suppress cell aggregation include a culture under conditions in which the culture medium flows such that cells are concentrated on a spot due to stress (centrifugal force, centripetal force) caused by a flow such as a swirling and/or rocking flow; and a culture under conditions in which the culture medium flows due to a linear back and forth movement, and particularly preferred is a culture by a swirling culture method or a rocking culture method.

The "swirling culture method" (including shaking culture method) refers to a method of culturing under conditions in which the culture medium flows such that cells are concentrated on a spot due to stress (centrifugal force, centripetal force) caused by a swirling flow. Specifically, the swirling culture method is carried out by swirling a cultureware including a culture medium containing cells in a manner to draw a closed orbit such as a circle, ellipse, flattened circle or flattened ellipse along generally a horizontal plane, or by swirling a culture medium in a cultureware with a stirrer such as a stirrer bar or stirrer blade while the cultureware is left standing. The latter may be accomplished by using, for example, a spinner flask-like cultureware with agitator blades. Such culturewares are commercially available and the commercial products may be used. In that case, the volume of the culture medium, culture solution or the like may be set as recommended by the cultureware manufacturer.

The speed of swirling in the swirling culture method is not particularly limited, and the upper limit may be, for example, 200 rpm or less, 150 rpm or less, 120 rpm or less, 115 rpm or less, 110 rpm or less, 105 rpm or less, 100 rpm or less, 95 rpm or less, or 90 rpm or less. The lower limit may be, for example, 1 rpm or more, 10 rpm or more, 50 rpm or more, 60 rpm or more, 70 rpm or more, 80 rpm or more, or 90 rpm or more. The swirling width during the swirling culture is not particularly limited, and the lower limit may be, for example, 1 mm or more, 10 mm or more, 20 mm or more, or 25 mm or more. The upper limit of the swirling width may be, for example, 200 mm or less, 100 mm or less, 50 mm or less, 30 mm or less, or 25 mm or less. The radius of rotation during the swirling culture is not particularly limited, and is set such that the swirling width is within the above-mentioned range. The lower limit of the radius of rotation may be, for example, 5 mm or more, or 10 mm or more, and the upper limit thereof may be, for example, 100 mm or more, or 50 mm or more. Setting the swirling culture condition to these ranges is preferable because it becomes easy to produce cell aggregates with an appropriate size.

The "rocking culture method" refers to a method of culturing under conditions that a rocking flow is imparted to a culture medium by a linear reciprocating motion such as rocking agitation. Specifically, the rocking culture method is carried out such that a cultureware including a culture medium containing cells is rocked in a plane generally vertical to a horizontal plane. The speed of rocking is not particularly limited, and for example, when one round trip is set as one time, the rocking may be carried out with the lower limit of 2 times or more, 4 times or more, 6 times or more, 8 times or more, or 10 times or more per minute, and the upper limit of 15 times or less, 20 times or less, 25 times or less, or 50 times or less per minute. During rocking, it is preferable to impart some angle relative to the vertical surface, i.e., rocking angle, to the cultureware. The rocking angle is not particularly limited, and, for example, the lower limit may be 0° or more, 1° or more, 2° or more, 4° or more, 6° or more, or 8° or more, and the upper limit may be 10° or less, 12° or less, 15° or less, 18° or less, or 20° or less. Setting the rocking culture condition to these ranges is preferable because cell aggregates with an appropriate size can be produced.

Further, the culture may be mixed by movement in which the above rotary shaking and rocking are combined.

Culture using spinner flask-shaped cultureware in which mixing blades are placed may be carried out. During this culture, the liquid culture medium is mixed by the mixing blades. The speed of rotation and the volume of culture medium are not particularly limited. When a commercially available spinner flask-shaped cultureware is used, the volume recommended by the manufacturer may be suitably used as a volume of cell culture composition. The speed of rotation has no particular limitation and may be, for example, 10 rpm or more and 100 rpm or less.

The seeding density (i.e., the cell density at the start of suspension culture) of cells cultured in suspension in a liquid culture medium may be adjusted appropriately. The lower limit is, for example, 0.01 × 10⁵ cells/mL or more, 0.1 × 10⁵ cells/mL or more, and 1 × 10⁵ cells/mL or more. The upper limit of the seeding density is, for example, 10× 10⁶ cells/mL or less, 20 × 10⁵ cells/mL or less, or 10 × 10⁵ cells/mL or less. When the seeding density is within this range, cell aggregates with an appropriate size are likely to be formed. For example, the seeding density may be 0.1 × 10⁵ cells/mL, 0.2 × 10⁵ cells/mL, 0.3 × 10⁵ cells/mL, 0.4 × 10⁵ cells/mL, 0.5 × 10⁵ cells/mL, 0.6 × 10⁵ cells/mL, 0.7 × 10⁵ cells/mL, 0.8 × 10⁵ cells/mL, 0.9 × 10⁵ cells/mL, 1 × 10⁵ cells/mL, 1.5 × 10⁵ cells/mL, 2 × 10⁵ cells/mL, 3 × 10⁵ cells/mL, 4 × 10⁵ cells/mL, 5 × 10⁵ cells/mL, 6 × 10⁵ cells/mL, 7 × 10⁵ cells/mL, 8 × 10⁵ cells/mL, 9 × 10⁵ cells/mL, or 10 × 10⁶ cells/mL.

The volume of cell culture composition during suspension culture may be appropriately adjusted depending on the cultureware used. For example, when a 12-well plate (with a bottom area per well of 3.5 cm² in a flat view) is used, the volume may be 0.5 mL/well or more and 1.5 mL/well or less, and, for example, 1.3 mL/well. For example, when a 6-well plate (with a bottom area per well of 9.6 cm² in a flat view) is used, the volume may be 1.5 mL/well or more, for example, 2 mL/well or more, and 3 mL/well or more, and 6.0 mL/well or less, 5 mL/well or less, and 4 mL/well or less. When a 125-mL Erlenmeyer flask (an Erlenmeyer flask with a volume of 125 mL) is used, for example, the volume may be 10 mL/flask or more, 15 mL/flask or more, 20 mL/flask or more, 25 mL/flask or more, 20 mL/flask or more, 25 mL/flask or more, and 30 mL/flask or more. The volume may be 50 mL/flask or less, 45 mL/flask or less, and 40 mL/flask or less. When a 500-mL Erlenmeyer flask (an Erlenmeyer flask with a volume of 500 mL) is used, for example, the volume may be 100 mL/flask or more, 105 mL/flask or more, 110 mL/flask or more, 115 mL/flask or more, and 120 mL/flask or more. The volume may be 150 mL/flask or less, 145 mL/flask or less, 140 mL/flask or less, 135 mL/flask or less, 130 mL/flask or less, and 125 mL/flask or less. When a 1000-mL Erlenmeyer flask (an Erlenmeyer flask with a volume of 1000 mL) is used, for example, the volume may be 250 mL/flask or more, for example, 260 mL/flask or more, 270 mL/flask or more, 280 mL/flask or more, and 290 mL/flask or more. The volume may be 350 mL/flask or less, 340 mL/flask or less, 330 mL/flask or less, 320 mL/flask or less, and 310 mL/flask or less. When a 2000-mL Erlenmeyer flask (an Erlenmeyer flask with a volume of 2000 mL) is used, for example, the volume may be 500 mL/flask or more, 550 mL/flask or more, and 600 mL/flask or more. The volume may be 1000 mL/flask or less, 900 mL/flask or less, 800 mL/flask or less, and 700 mL/flask or less. When a 3000-mL Erlenmeyer flask (an Erlenmeyer flask with a volume of 3000 mL) is used, for example, the volume may be 1000 mL/flask or more, preferably 1100 mL/flask or more, 1200 mL/flask or more, 1300 mL/flask or more, 1400 mL/flask or more, and 1500 mL/flask or more. The volume may be 2000 mL/flask or less, 1900 mL/flask or less, 1800 mL/flask or less, 1700 mL/flask or less, and 1600 mL/flask or less. When a 2-L culture bag (a disposable culture bag with a volume of 2 L) is used, for example, the volume may be 100 mL/bag or more, 200 mL/bag or more, 300 mL/bag or more, 400 mL/bag or more, 500 mL/bag or more, 600 mL/bag or more, 700 mL/bag or more, 800 mL/bag or more, 900 mL/bag or more, and 1000 mL/bag or more. The volume may be 2000 mL/bag or less, 1900 mL/bag or less, 1800 mL/bag or less, 1700 mL/bag or less, 1600 mL/bag or less, 1500 mL/bag or less, 1400 mL/bag or less, 1300 mL/bag or less, 1200 mL/bag or less, and 1100 mL/bag or less. When a 10-L culture bag (a disposable culture bag with a volume of 10 L) is used, for example, the volume may be 500 mL/bag or more, 1 L/bag or more, 2 L/bag or more, 3 L/bag or more, 4 L/bag or more, and 5 L/bag or more. The volume may be 10 L/bag or less, 9 L/bag or less, 8 L/bag or less, 7 L/bag or less, and 6 L/bag or less. When a 20-L culture bag (a disposable culture bag with a volume of 20 L) is used, for example, the volume may be 1 L/bag or more, 2 L/bag or more, 3 L/bag or more, 4 L/bag or more, 5 L/bag or more, 6 L/bag or more, 7 L/bag or more, 8 L/bag or more, 9 L/bag or more, and 10 L/bag or more. The volume may be 20 L/bag or less, 19 L/bag or less, 18 L/bag or less, 17 L/bag or less, 16 L/bag or less, 15 L/bag or less, 14 L/bag or less, 13 L/bag or less, 12 L/bag or less, and 11 L/bag or less. When a 50-L culture bag (a disposable culture bag with a volume of 50 L) is used, for example, the volume may be 1 L/bag or more, 2 L/bag or more, 5 L/bag or more, 10 L/bag or more, 15 L/bag or more, 20 L/bag or more, and 25 L/bag or more. The volume may be 50 L/bag or less, 45 L/bag or less, 40 L/bag or less, 35 L/bag or less, and 30 L/bag or less. When the volume of cell culture composition is within these ranges, cell aggregates with an appropriate size are likely to be formed.

The volume of cultureware used has no particular limitation and may be suitably selected. The area of the bottom of a portion housing a liquid culture medium may be determined in a flat view. The lower limit of the bottom area of the cultureware used may be, for example, 0.32 cm² or more, 0.65 cm² or more, 0.65 cm² or more, 1.9 cm² or more, and 3.0 cm² or more, 3.5 cm² or more, 9.0 cm² or more, or 9.6 cm² or more. The upper limit of the bottom area of the cultureware used may be, for example, 1000 cm² or less, 500 cm² or less, 300 cm² or less, 150 cm² or less, 75 cm² or less, 55 cm² or less, 25 cm² or less, 21 cm² or less, 9.6 cm² or less, and 3.5 cm² or less.

Conditions such as the temperature, culture period, CO₂ level of cell suspension culture in the presence of the agonist of thrombin receptor are not particularly limited. The culture temperature is 20°C or higher and 35°C or higher and 45°C or lower, 40°C or lower, or 37°C. The culture period is 0.5 hour or longer and more 12 hours or longer and 7 days or shorter, 72 hours or shorter, 48 hours or shorter, or 24 hours or shorter. The CO₂ level during the culture is 4% or higher and 4.5% or higher and 10% or lower, 5.5% or lower, or 5%. Furthermore, in the methods for suppressing cell aggregation of the present invention, a passage procedure may be accompanied during the suspension culture step. When the culture conditions are within these ranges, cell aggregates with an appropriate size are likely to be formed. Furthermore, the culture medium can be exchanged in an appropriate frequency. The frequency of the culture medium exchange may vary depending on the cell species to be cultured, but, for example, may be one or more times every 5 days, one or more times every 4 days, one or more times every 3 days, one or more times every 2 days, one or more times a day. The culture medium exchange may be carried out by collecting cells in the same manner as in the collection step described below, then adding a fresh culture medium, subsequently gently dispersing cell aggregates; and then culturing again.

In the suspension culture step, to what extent the number of cells is increased and to which the state of cells is to be met may be determined appropriately depending on the type of cells to be cultured, the purpose of cell aggregation, the type of culture medium, and the culture conditions. It is preferable that the cells used in the suspension culture step are cells cultured by a culture step in advance, then collected by a collection step, and single-cellularized as needed. The maintenance culture step, collection step, and single-cellularization are as described below. After the suspension culture step, the culture solution is discarded by a common procedure and the cells are collected. At this time, the cells are preferably collected as single cells by a detachment or dispersion treatment. Specific methods thereof are described in detail in the collection step described below. The collected cells may be directly, or after being washed with a buffer (including a PBS buffer), saline, or culture medium (preferably a culture medium used in the next step or a basal medium) as needed, subjected to the next step.

### (Maintenance Culture Step)

A "maintenance culture step" is a step of culturing a cell population before a suspension culture step, or a cell aggregate obtained after a suspension culture step or after a subsequent collection step to proliferate the cells while remaining undifferentiated. The maintenance culture may be adherent culture in which cells are cultured while adhered to a culture substrate such as a container or a support, or may be suspension culture in which cells are cultured in suspension in a culture medium.

In the maintenance culture step, cells of interest may be cultured by known animal cell culture methods in the art. The culture in the maintenance culture step may be adhesion culture or suspension culture.

Specific embodiments of the culture medium and cells used in the maintenance culture step are as described above.

The cultureware, seeding density of cells, and culture conditions used in the maintenance culture step are as described above with respect to the suspension culture step.

The flow state of the culture medium in the maintenance culture step is not limited. The maintenance culture may be static culture or fluid culture.

The "static culture" refers to culturing cells while standing the culture medium in a cultureware. In adhesion culture, this static culture is typically employed.

The "fluid culture" refers to culturing cells under conditions in which the culture medium is flowed. Specific embodiments of the fluid culture are as described above with respect to the suspension culture step.

In the maintenance culture step, to what extent the number of cells is increased and to which the state of cells is to be met may be determined appropriately depending on the type of cells to be cultured, the purpose of cell aggregation, the type of culture medium, and the culture conditions.

A suitable aspect of the maintenance culture step is a maintenance culture step that further cultures cell aggregates formed by a suspension culture step in the presence of an agonist of thrombin receptor. The method of culture in the maintenance culture step in this aspect is not particularly limited, and examples thereof include a step of culturing cell aggregates in suspension in a culture medium free of agonists of thrombin receptor. As the culture medium used for this maintenance culture, the same culture medium as described above except that agonists of thrombin receptor are not contained can be used. As the conditions for the maintenance culture, the same conditions as those in the suspension culture step can be used. In the maintenance culture step in this aspect, it is preferable that the culture medium is exchanged in an appropriate frequency. The frequency of the medium change may vary depending on a type of the cells. The frequency of medium change operation may be, for example, once or more per 5 days, once or more per 4 days, once or more per 3 days, once or more per 2 days, or once or more per day. This frequency of the culture medium exchange is particularly suitable when cell aggregates of stem cells are cultured. Methods of the culture medium exchange are not particularly limited, and a preferable method may include: collecting all the volume of the cell aggregate-containing cell culture composition into a centrifuge tube; subjecting the tube to centrifugation or a standing state for about 5 minutes; removing the supernatant from precipitated cell aggregates; then adding a fresh culture medium; gently dispersing the cell aggregates, and then returning the cell aggregate-dispersed culture medium to a cultureware such as a plate, so that the cell aggregates can be cultured continuously. The culture period of the maintenance culture step in this aspect is not particularly limited, and preferably 3 days or more and 7 days or less. By further culturing the cell aggregates in suspension in a culture medium free of agonists of thrombin receptor in the conditions described above, cell aggregates with an appropriate size can be obtained.

After the maintenance culture step, the culture solution is discarded by a common procedure and the cells are collected. At this time, the cells are preferably collected as single cells by a detachment or dispersion treatment. Specific methods thereof are described in detail in the collection step described below. The collected cells may be directly, or after being washed with a buffer (including a PBS buffer), saline, or culture medium (preferably a culture medium used in the next step or a basal medium) as needed, subjected to the next step.

### (Collection Step)

The "collection step" is a step of collecting cultured cells from a culture solution after a suspension culture step and/or a maintenance culture step, and is an optional step in the method of the present invention.

As used herein, "collection (of cells)" refers to separating cells from a culture solution to obtain the cells. The collection method of cells may follow a common procedure used in cell culture methods in the art, and is not particularly limited. The cell culture methods can generally be classified into suspension culture methods and adhesion culture methods. Hereinafter, the collection method of cells after each culture method will be described.

### (Collection Method After Suspension Culture Method)

When cultured in a suspension culture method, the cells are present in a suspension state in the culture solution. Thus, collection of cells can be accomplished by removing liquid components of the supernatant in the static state or by centrifugation. Furthermore, filters, hollow filament separation membranes or the like can be selected as collection methods of cells. In the case of removing liquid components in the static state, the container containing the culture solution may be left in the static state for about 5 minutes, and the supernatant may be removed to leave the deposited cells or cell aggregates. Centrifugation may also be performed at a rotational speed and processing time at which cells are not damaged by centrifugal forces. For example, the lower limit of the rotational speed is not particularly limited as long as the cells can be deposited, and may be, for example, 500 rpm or more, 800 rpm or more, or 1000 rpm or more. While, the upper limit is not limited as long as the cells do not suffer or are not vulnerable to damage by centrifugal forces at the rotational speed, and may be, for example, 1400 rpm or less, 1500 rpm or less, or 1600 rpm or less. The lower limit of the processing time is not particularly limited as long as the cells can be deposited at the rotational speed, and may be, for example, 30 seconds, 1 minute, 3 minutes, or 5 minutes. The upper limit is not limited as long as the cells do not or hardly suffer from damage by the rotation, and may be, for example, 30 seconds, 6 minutes, 8 minutes, or 10 minutes. The collected cells can be washed as needed. Methods for washing are not limited. For example, the same methods as the washing method described in "treatment after the step" in the maintenance culture step described above can be used. A buffer (including PBS buffer), saline, or culture medium (preferably, basal medium) may be used as a washing solution.

### (Collection Method After Adhesion Culture Method)

When cultured in an adhesion culture method, many cultured cells are present while adhered to an external matrix such as a cultureware and a culture support. Thus, to remove the culture solution from the cultureware, the cultureware may be gently tilted after the culture to drain liquid components. Since the cells adhered to the external matrix remain in the cultureware, the cells and the culture solution can be readily separated.

Cell surfaces adhered to the external matrix can then be washed as needed. A buffer (including a PBS buffer), saline, or culture medium (preferably, basal medium) may be used as a washing solution. However, the washing solution is not limited thereto. The washing solution after washing may be removed in the same manner as the culture solution. This washing step may be repeated multiple times.

The cell population adhered to the external matrix is then detached from the external matrix. The detachment method may be performed in a manner known in the art. Typically, scraping, detaching agents containing proteolytic enzymes as active ingredients, chelating agents such as EDTA, or mixtures of detaching agents and chelating agents, or the like are used.

Scraping is a method for stripping cells attached to an external matrix by mechanical means such as scrapers. However, since cells are vulnerable to damage by mechanical procedures, when the collected cells are subjected to further culture, it is preferable to employ a detachment method which chemically destroys or degrades the scaffold portion of cells bound to an external matrix and releases the adhesion between the cells and the external matrix.

In the detachment method, a detaching agent and/or a chelating agent are used. The detaching agent is not limited, and examples thereof include trypsin, collagenase, pronase, hyaluronidase, elastase, as well as commercially available Accutase (registered trade mark), TrypLE™ Express Enzyme (Life Technologies Japan Ltd.), TrypLE™ Select Enzyme (Life Technologies Japan Ltd.), "Dispase" (registered trade mark). The concentration and processing time of each detaching agent may be set in the range of those in common procedures for cell detachment or dispersion. For example, when the detaching agent is trypsin, the lower limit of the concentration in the solution is not particularly limited as long as the cells can be detached at the concentration, and may be, for example, 0.01% or more, 0.02% or more, 0.03% or more, 0.04% or more, 0.05% or more, 0.08% or more, or 0.10% or more. The upper limit of the concentration in the solution is not particularly limited as long as cells themselves are not affected with lysis or the like by the action of trypsin at the concentration, and may be, for example, 0.15% or less, 0.20% or less, 0.25% or less, or 0.30% or less. The processing time also depends on the concentration of trypsin, but the lower limit is not particularly limited as long as the cells can be sufficiently detached from the external matrix by the action of trypsin at the time, and may be, for example, 1 minute or more, 2 minutes or more, 3 minutes or more, 4 minutes, or 5 minutes or more. The upper limit of the processing time is not particularly limited as long as cells themselves are not affected with lysis or the like by the action of trypsin at the time, and may be, for example, 8 minutes or less, 10 minutes or less, 12 minutes or less, 15 minutes or less, 18 minutes or less, or 20 minutes or less. Other detaching agents and chelating agents can be used generally in the same manner as described above. When commercially available detaching agents are used, the concentrations and processing times described in the attached protocol can be employed.

The cells detached from the external matrix are separated from the supernatant containing detaching agents by centrifugation. The centrifugal conditions may be the same as those in the "Collection Method After Suspension Culture Method" described above. The collected cells can be washed as needed. The washing method may also be carried out in the same manner as those in the "Collection Method After Suspension Culture Method" described above.

The cells obtained after this step may partially include cell assemblies such as monolayer cell fragments and cell aggregates. The collected cells can be single-cellularized as needed.

### (Single-cellularization)

As used herein, "single-cellularization" refers to dispersing cell assemblies such as monolayer cell fragments and cell aggregates in which multiple cells are adhered or aggregated each other to make a state of single free cells.

Single-cellularization can be performed by increasing the concentration of detaching agents and/or chelating agents and/or by extending the processing time with detaching agents and/or chelating agents used in the above-described detachment method. For example, when the detaching agent is trypsin, the lower limit of the concentration in the solution is not particularly limited as long as cell assemblies can be dispersed at the concentration, and may be, for example, 0.15% or more, 0.18% or more, 0.20% or more, or 0.24% or more. The upper limit of the concentration in the solution is not particularly limited as long as the cells themselves are not affected with lysis or the like at the concentration, and may be 0.25% or less, 0.28% or less, or 0.30% or less. The processing time also depends on the concentration of trypsin, but the lower limit is not particularly limited, as long as cell assemblies can be sufficiently dispersed by the action of trypsin at the time, and may be, for example, 5 minutes or more, 8 minutes or more, 10 minutes or more, 12 minutes or more, or 15 minutes or more. The upper limit of the processing time is not particularly limited as long as the cells themselves are not affected with lysis or the like by the action of trypsin at the time, and may be, for example, 18 minutes or less, 20 minutes or less, 22 minutes or less, 25 minutes or less, 28 minutes or less, or 30 minutes or less. When commercially available detaching agents are used, the agent may be used at the concentration at which the cells can be dispersed to be a single state as described in the attached protocol. Single-cellularization can be facilitated by physically lightly treating cells after treating with the detaching agent and/or chelating agent. This physical treatment is not limited, and examples thereof include a method of pipetting cells together with the solution multiple times. Additionally, cells may be passed through a strainer or mesh as needed.

Single-cellularized cells can be collected by removing supernatants containing detaching agents by standing or centrifugation. The collected cells may be washed as needed. Conditions for centrifugation and methods for washing can be carried out in the same manner as in the "Collection Method After Suspension Culture Method" described above.

### <6. Cell Aggregation Suppressor>

Another aspect of the present invention is a cell aggregation suppressor for use in suspension culture of cells, comprising an agonist of a thrombin receptor.

The cell aggregation suppressor of the present invention can be used to appropriately suppress aggregation of cells in suspension culture to form cell aggregates with a substantially uniform size. Furthermore, in suspension culture of stem cells using the cell aggregation suppressor of the present invention, the stem cells can remain undifferentiated.

The form of the cell aggregation suppressor according to the present invention is not particularly limited, and may be the agonist of a thrombin receptor itself, or a composition of the agonist of a thrombin receptor in combination with other components. The form of the composition is not particularly limited. The composition may be, for example, a form of a culture medium used for suspension culture or may be a form of an additive composition mixed when a culture medium is prepared.

A preferred embodiment of the cell aggregation suppressor according to the present invention is a culture medium or a buffer such as a phosphate buffer comprising the agonist of a thrombin receptor. Examples of the concentration of the agonist of a thrombin receptor in the culture medium include the concentration of the agonist of a thrombin receptor in a culture medium described for suspension culture in the column <5. Methods for Suppressing Aggregation of Cells>.

Another preferred embodiment of the cell aggregation suppressor according to the present invention is a liquid or solid composition comprising the agonist of a thrombin receptor in a liquid or solid medium. The liquid or solid composition is an additive which is added when a culture medium for suspension culture is prepared. It is preferable that the cell aggregation suppressor of this embodiment is prepared such that the final concentration of the agonist of a thrombin receptor in the culture medium to be prepared is a concentration of the agonist of a thrombin receptor in the culture medium described for suspension culture in the column <5. Methods for Suppressing Aggregation of Cells>. When the cell aggregation suppressor is a composition, the lower limit of the concentration of the agonist of a thrombin receptor in the cell aggregation suppressor is not particularly limited as long as the effect as an cell aggregation suppressor is exhibited, and is, for example, 1-fold or more, 2-fold or more, 10-fold or more, 100-fold or more, 1000-fold or more, or 10000-fold or more of the concentration of the agonist of a thrombin receptor described above as the preferred concentration in the culture medium during suspension culture. Specifically, it is in the range of 7.4 µg/mL or more and 38 mg/mL or less, and the lower limit may be 10 µg/mL or more, 20 µg/mL or more, 30 µg/mL or more, 38 µg/mL or more, 50 µg/mL or more, 100 µg/mL or more, 150 µg/mL or more, 200 µg/mL or more, 250 µg/mL or more, 300 µg/mL or more, 350 µg/mL or more, or 380 µg/mL or more. The upper limit is not particularly limited as long as the cells are not killed at the concentration and the agonist of a thrombin receptor is soluble in the solvent, and examples thereof include 38 mg/mL or less, 30 mg/mL or less, 20 mg/mL or less, 10 mg/mL or less, 9 mg/mL or less, 8 mg/mL or less, 7 mg/mL or less, 6 mg/mL or less, 5 mg/mL or less, 4 mg/mL or less, and 3.8 mg/mL or less. Alternatively, examples of the concentration of the agonist of a thrombin receptor include 10 µM or more and 50 mM or less. The lower limit of the concentration is, for example, 10 µM or more, 20 µM or more, 30 µM or more, 40 µM or more, 50 µM or more, 100 µM or more, 200 µM or more, 300 µM or more, 400 µM or more, 500 µM or more, 600 µM or more, 700 µM or more, 800 µM or more, 900 µM or more, 1 mM or more, 2 mM or more, 3 mM or more, 4 mM or more, or 5 mM or more, and the upper limit thereof may be 50 mM or less, 45 mM or less, 40 mM or less, 35 mM or less, 30 mM or less, 25 mM or less, 20 mM or less, 15 mM or less, 10 mM or less, or 5 mM or less. Compositions comprising the agonist of a thrombin receptor at the concentration within the above-mentioned range are easy to mix with other medium components to prepare a culture medium comprising the agonist of a thrombin receptor within the appropriate concentration range described for suspension culture in the column <5. Methods for Suppressing Aggregation of Cells>.

In addition to the agonist of a thrombin receptor, the cell aggregation suppressor may contain, as additives, antibiotics, kinase inhibitor buffers, thickeners, colorants, stabilizers, surfactants, emulsifiers, preservatives, preserving agents, antioxidants, and the like. Examples of the antibiotics that can be used include, but are not particularly limited to, penicillin, streptomycin, and amphotericin B. Preferable examples of the kinase inhibitors include, but are not particularly limited to, ROCK inhibitors. Preferable examples of the ROCK inhibitors include, but are not particularly limited to, Y-27632. When Y-27632 is included, the concentration of Y-27632 in the cell aggregation suppressor is preferably prepared such that the final concentration of Y-27632 in the culture medium to be prepared is a concentration of Y-27632 in the culture medium described for suspension culture in the column <5. Methods for Suppressing Aggregation of Cells>. The concentration of Y-27632 in the cell aggregation suppressor is not particularly limited, and is, for example, 1-fold or more, 2-fold or more, 10-fold or more, 100-fold or more, 1000-fold or more, or 10000 fold or more of the concentration of Y-27632 described above as the preferred concentration in the culture medium during suspension culture, and specifically may be 6.7 µg/mL or more and 14.0 mg/mL or less. The lower limit of the concentration of Y-27632 in the cell aggregation suppressor is not particularly limited as long as the effect is exhibited at the concentration, and for example, 6.7 µg/mL or more, 67 µg/mL or more, 670 µg/mL or more, 1 mg/mL or more, 2 mg/mL or more, 3 mg/mL or more, 4 mg/mL or more, 5 mg/mL or more, or 6 mg/mL or more. The upper limit is not particularly limited as long as the cells are not killed at the concentration and Y-27632 is soluble in the solvent, and is, for example, 14.0 mg/mL or less, or 10 mg/mL or less, or may be 6.7 mg/mL. Alternatively, examples of the concentration of Y-27632 include 20 µM or more and 40 mM or less. The lower limit of the concentration is not particularly limited as long as the effect is exhibited at the concentration, and for example, 20 µM or more, 0.2 mM or more, 0.3 mM or more, 0.4 mM or more, 0.5 mM or more, 0.6 mM or more, 0.7 mM or more, 0.8 mM or more, 0.9 mM or more, 1 mM or more, 2 mM or more, 3 mM or more, 4 mM or more, 5 mM or more, 6 mM or more, 7 mM or more, 8 mM or more, 9 mM or more, 10 mM or more, 11 mM or more, 12 mM or more, 13 mM or more, 14 mM or more, 15 mM or more, 16 mM or more, 17 mM or more, 18 mM or more, 19 mM or more, or 20 mM or more. The upper limit thereof is, for example, 40 mM or less, or 30 mM or less. Examples of the buffer include a phosphate buffer, tris-hydrochloric acid buffer, and glycine buffer. Examples of the thickener include gelatin and polysaccharides. Examples of the colorant include Phenol Red. Examples of the stabilizer include albumin, dextran, methyl cellulose, and gelatin. Examples of the surfactant include cholesterol, an alkyl glycoside, alkyl polyglycoside, alkyl monoglyceride ether, glucoside, maltoside, neopentyl glycol series, polyoxyethylene glycol series, thioglucoside, thiomaltoside, peptide, saponin, phospholipid, sorbitan fatty acid ester, and fatty acid diethanolamide. Examples of the emulsifier include a glycerin fatty acid ester, sorbitan fatty acid ester, propylene glycol fatty acid ester, and sucrose fatty acid ester. Examples of the preservative include aminoethyl sulfonic acid, benzoic acid, sodium benzoate, ethanol, sodium edetate, agar, dl-camphor, citric acid, sodium citrate, salicylic acid, sodium salicylate, phenyl salicylate, dibutylhydroxy toluene, sorbic acid, potassium sorbate, nitrogen, dehydro acetic acid, sodium dehydroacetate, 2-naphthol, white soft sugar, honey, paraoxy isobutyl benzoate, paraoxy isopropyl benzoate, paraoxy ethyl benzoate, paraoxy butyl benzoate, paraoxy propyl benzoate, paraoxy methyl benzoate, 1-menthol, and eucalyptus oil. Examples of the preserving agent include benzoic acid, sodium benzoate, ethanol, sodium edetate, dried sodium sulfite, citric acid, glycerin, salicylic acid, sodium salicylate, dibutylhydroxy toluene, D-sorbitol, sorbic acid, potassium sorbate, sodium dehydroacetate, paraoxy isobutyl benzoate, paraoxy isopropyl benzoate, paraoxy ethyl benzoate, paraoxy butyl benzoate, paraoxy propyl benzoate, paraoxy methyl benzoate, propylene glycol, and phosphoric acid. Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, super oxide dismutase (SOD), glutathione peroxidase, glutathione-S-transferase, glutathione reductase, catalase, ascorbic acid peroxidase, and mixtures thereof.

The cell aggregation suppressor may contain a growth factor, preferably one or more growth factors of FGF2 and TGF-β1.

### <7. Method for Producing Cell Aggregates, and Cell Aggregates Produced Thereby>

Another aspect of the present invention is a method for producing cell aggregates, comprising a step of culturing cells in suspension in a culture medium comprising an agonist of a thrombin receptor at a concentration of 3.7 ng/mL or more and 3.8 mg/mL or less, or 5 nM or more and 5 mM or less.

According to this method, cell aggregates with appropriate size can be produced in high yields. In particular, when the cells are stem cells, cell aggregates with appropriate size in which the stem cells remain undifferentiated can be produced in high yields.

Specific embodiments of the agonist of thrombin receptor, culture medium, and cell are as described above.

The lower limit of the concentration of the agonist of thrombin receptor in the culture medium in the step described above is not particularly limited as long as the effect as a cell aggregation suppressor is exerted, and is, for example, 3.7 ng/mL or more, 5 ng/mL or more, 10 ng/mL or more, and 15 ng/mL or more, 20 ng/mL or more, 25 ng/mL or more, 30 ng/mL or more, 35 ng/mL or more, 37 ng/mL or more, 50 ng/mL or more, 100 ng/mL or more, 150 ng/mL or more, 200 ng/mL or more, 250 ng/mL or more, 300 ng/mL or more, 350 ng/mL or more, 370 ng/mL or more, 400 ng/mL or more, 500 ng/mL or more, 600 ng/mL or more, 700 ng/mL or more, 800 ng/mL or more, 900 ng/mL or more, or 925 ng/mL or more. The upper limit is not particularly limited as long as the cells are not to be killed at the concentration, and is, for example, 3.8 mg/mL or less, 3 mg/mL or less, and 2 mg/mL or less, 1 mg/mL or less, 900 µg/mL or less, 800 µg/mL or less, 700 µg/mL or less, 600 µg/mL or less, 500 µg/mL or less, 400 µg/mL or less, 380 µg/mL or less, 300 µg/mL or less, 200 µg/mL or less, 100 µg/mL or less, 50 µg/mL or less, 38 µg/mL or less, or 15.2 µg/mL or less. The lower limit of the concentration of the agonist of thrombin receptor in the culture medium in the step described above is, for example, 5 nM or more, 50 nM or more, 0.1 µM or more, 0.5 µM or more, 0.6 µM or more, 0.7 µM or more, 0.8 µM or more, 0.9 µM or more, 1 µM or more, 1.25 µM or more, 2 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, or 20 µM or more. The upper limit is, for example, 5 nM or less, 500 µM or less, 50 µM or less, or 20 µM or less. When the concentration of the agonist of a thrombin receptor in the culture medium is within the above-mentioned range, suppression on aggregation of cells is effected without affecting undifferentiated states of the cells or the like, and the size of the cell aggregate can be regulated.

Specific embodiments of the step are similar to the specific embodiments of the "step of culturing cells in suspension in a culture medium comprising an agonist of a thrombin receptor" in the method for suppressing aggregation of cells described in the column <5. Methods for Suppressing Aggregation of Cells>.

Another aspect of the present invention is a cell aggregate produced by the method for producing a cell aggregate described above.

The cell aggregate according to this aspect of the present invention has an appropriate size and the cells constituting the cell aggregate have high viable cell ratio. Furthermore, when the cell are stem cells, the cells constituting the cell aggregate remain undifferentiated.

The cell aggregate according to this aspect of the present invention preferably has the features described in the column <2. Cell Aggregates>.

The method for producing a cell aggregate of the present invention can also appropriately comprise an optional step, in addition to a suspension culture step of culturing cells in suspension in a culture medium comprising an agonist of a thrombin receptor. Examples of the optional step include a maintenance culture step and a collection step of cell aggregates. Furthermore, the suspension culture may include a passage procedure. Suitable embodiments of the maintenance culture step and the collection step are similar to the maintenance culture step and the collection step described in the column <5. Methods for Promoting Cell Aggregation>.

### <8. Cell Culture Composition>

Another aspect of the present invention is a cell culture composition comprising cells, a culture medium, and an agonist of a thrombin receptor at a concentration of 3.7 ng/mL or more and 3.8 mg/mL or less, or 5 nM or more and 5 mM or less.

The cell culture composition according to this aspect of the present invention can be used to produce cell aggregates in high yields. In particular, when the cells are stem cells, the cell culture composition can be used to produce cell aggregates with appropriate size, in which the stem cells remain undifferentiated, in high yields.

Specific embodiments of the agonist of a thrombin receptor, culture medium, and cell are as described above.

The lower limit of the concentration of the agonist of a thrombin receptor in the cell culture composition according to this aspect of the present invention is not particularly limited as long as the effect as an cell aggregation suppressor is exhibited, and for example, 3.7 ng/mL or more, 3.7 ng/mL or more, 5 ng/mL or more, 10 ng/mL or more, 15 ng/mL or more, 20 ng/mL or more, 25 ng/mL or more, 30 ng/mL or more, 35 ng/mL or more, 37 ng/mL or more, 50 ng/mL or more, 100 ng/mL or more, 150 ng/mL or more, 200 ng/mL or more, 250 ng/mL or more, 300 ng/mL or more, 350 ng/mL or more, 370 ng/mL or more, 400 ng/mL or more, 500 ng/mL or more, 600 ng/mL or more, 700 ng/mL or more, 800 ng/mL or more, 900 ng/mL or more, or 925 ng/mL or more. The upper limit is not particularly limited as long as the cells are not to be killed at the concentration, and for example, 3.8 mg/mL or less, 3 mg/mL or less, 2 mg/mL or less, 1 mg/mL or less, 900 µg/mL or less, 800 µg/mL or less, 700 µg/mL or less, 600 µg/mL or less, 500 µg/mL or less, 400 µg/mL or less, 380 µg/mL or less, 300 µg/mL or less, 200 µg/mL or less, 100 µg/mL or less, 50 µg/mL or less, 38 µg/mL or less, or 15.2 µg/mL or less. The lower limit of the concentration of the agonist of a thrombin receptor in the cell culture compositions according to this aspect of the present invention is, for example, 5 nM or more, 50 nM or more, 0.1 µM or more, 0.5 µM or more, 0.6 µM or more, 0.7 µM or more, 0.8 µM or more, 0.9 µM or more, 1 µM or more, 1.25 µM or more, 2 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, or 20 µM or more. The upper limit is, for example, 5 mM or less, 500 µM or less, 50 µM or less, or 20 µM or less. The cell culture compositions in which the concentration of the agonist of a thrombin receptor is within the above-mentioned range are particularly suitable for producing cell aggregates with an appropriate size in high yields.

Examples of the step of producing a cell aggregate from the cell culture composition described above include a step of culturing a cell in suspension in the cell culture composition described above. Specific embodiments of this step are similar to the specific embodiments of the "step of culturing cells in suspension in a culture medium comprising an agonist of a thrombin receptor" in the method for suppressing aggregation of cells described in the column

### <5. Methods for Suppressing Aggregation of Cells>.

The cell culture composition may be prepared by adding an agonist of a thrombin receptor to a culture medium, then adding cells to the culture medium, or by mixing cells with a culture medium, then adding an agonist of a thrombin receptor to the mixture. Preferably, the cell culture composition is prepared by adding an agonist of a thrombin receptor to a culture medium, then adding cells to the culture medium. A stabilizer can also be added when adding the agonist of a thrombin receptor to the culture medium. The stabilizer is not particularly limited as long as it is a substance that contributes to, for example, stabilization of the agonist of a thrombin receptor in a liquid culture medium, maintenance of the activity, and prevention of adsorption to the cultureware or the like, and examples thereof include proteins such as albumin, an emulsifier, a surfactant, an amphiphilic substance, and a polysaccharide compound such as heparin.

The cell culture composition may be prepared by freezing and storing a culture medium comprising an agonist of a thrombin receptor (optionally further comprising the stabilizer), and later thawing the culture medium and adding cells thereto.

### <9. Cell Culture Medium>

Another aspect of the present invention is a cell culture medium comprising a culture medium, and an agonist of a thrombin receptor at a concentration of 3.7 ng/mL or more and 3.8 mg/mL or less, or 5 nM or more and 5 mM or less.

The cell culture medium according to this aspect of the present invention can be used as a culture medium for producing cell aggregates from cells by suspension culture in high yields. In particular, when the cells are stem cells, the cell culture medium can be used to produce cell aggregates with appropriate size, in which the stem cells remain undifferentiated, in high yields.

Specific embodiments of the agonist of a thrombin receptor, culture medium, and cell are as described above.

The concentration of the agonist of a thrombin receptor in the cell culture medium according to this aspect of the present invention is, for example, 3.7 ng/mL or more, 5 ng/mL or more, 10 ng/mL or more, 15 ng/mL or more, 20 ng/mL or more, 25 ng/mL or more, 30 ng/mL or more, 35 ng/mL or more, 37 ng/mL or more, 50 ng/mL or more, 100 ng/mL or more, 150 ng/mL or more, 200 ng/mL or more, 250 ng/mL or more, 300 ng/mL or more, 350 ng/mL or more, 370 ng/mL or more, 400 ng/mL or more, 500 ng/mL or more, 600 ng/mL or more, 700 ng/mL or more, 800 ng/mL or more, 900 ng/mL or more, or 925 ng/mL or more. The upper limit is not particularly limited as long as the cells are not to be killed at the concentration, and is, for example, 3.8 mg/mL or less, 3 mg/mL or less, 2 mg/mL or less, 1 mg/mL or less, 900 µg/mL or less, 800 µg/mL or less, 700 µg/mL or less, 600 µg/mL or less, 500 µg/mL or less, 400 µg/mL or less, 380 µg/mL or less, 300 µg/mL or less, 200 µg/mL or less, 100 µg/mL or less, 50 µg/mL or less, 38 µg/mL or less, or 15.2 µg/mL or less, or 9.5 µg/mL or less. The concentration of the agonist of a thrombin receptor in the cell culture compositions according to this aspect of the present invention is, for example, 5 nM or more, 50 nM or more, 0.1 µM or more, 0.5 µM or more, 0.6 µM or more, 0.7 µM or more, 0.8 µM or more, 0.9 µM or more, 1 µM or more, 1.25 µM or more, 2 µM or more, 3 µM or more, 4 µM or more, 5 µM or more, 6 µM or more, 7 µM or more, 8 µM or more, 9 µM or more, 10 µM or more, 11 µM or more, 12 µM or more, 13 µM or more, 14 µM or more, 15 µM or more, 16 µM or more, 17 µM or more, 18 µM or more, 19 µM or more, or 20 µM or more. The upper limit is, for example, 5 mM or less, 500 µM or less, 50 µM or less, or 20 µM or less. The cell culture compositions in which the concentration of the agonist of a thrombin receptor is within the above-mentioned range are particularly suitable for producing cell aggregates with an appropriate size in high yields.

Examples of the step of producing a cell aggregate from the cell culture medium described above include a step of culturing cells in suspension in the cell culture medium described above. Specific embodiments of this step are similar to the specific embodiments of the "step of culturing cells in suspension in a culture medium comprising an agonist of a thrombin receptor" in the method for suppressing aggregation of cells described in the column

### <5. Methods for Suppressing Aggregation of Cells>.

The cell culture medium can be frozen and stored until use and thawed upon use.

### Examples

### <Example 1: Maintenance Culture of Human iPS Cells>

TkDN4-M cell lines (Institute of Medical Science, The University of Tokyo) were used as human iPS cells. Human iPS cells were seeded on cell culture dishes coated with Vitronectin (Thermo Fisher Scientific Co., Ltd.) and subjected to a maintenance culture using Essential 8™ (Thermo Fisher Scientific Co., Ltd.) as the culture medium. Accutase (Thermo Fisher Scientific Co., Ltd.) was used as a cell detachment agent during passage. In addition, when the cells were seeded, Y-27632 (Wako Pure Chemical Industries, Ltd.) at a concentration of 10 µM was added to a culture medium. The culture medium was changed every day. For experiments, human iPS cells (the number of passage was 50 or less) were used.

### <Example 2: Confirmation of Aggregation Suppression Effect by Cell Aggregation Assay>

The cell aggregation suppression effect by addition of agonists of a thrombin receptor was investigated.

### (Protocol)

Human iPS cells that had been cultured using the protocol of Example 1 were treated with Accutase for 3 to 5 minutes and were detached and dispersed to single cells. The resulting cells were suspended in Essential 8™ culture medium comprising a final concentration of 5 mg/mL of BSA (Wako Pure Chemical Industries, Ltd.) and 10 µM of Y-27632 (Wako Pure Chemical Industries, Ltd.), and a portion thereof was stained with trypan blue and the number of cells was counted. The cell suspension was prepared so as to contain 2 × 10⁵ cells per mL. Separately, the cell aggregation suppressor was adjusted so that the final concentration of the agonist of a thrombin receptor (Thrombin Receptor Activator for Peptide 6; TRAP-6, ANASPEC, AS-24191) was 3.74 mg/mL (10 mM), then the adjusted cell aggregation suppressor was added to the cell suspension so that the final concentration of the agonist of a thrombin receptor was 2 µM, 10 µM, or 50 µM. Then the cells were seeded at a ratio of 1.3 mL/well in a 12-well plate for suspension culture (Sumitomo Bakelite Co., Ltd.). The cell-seeded plate was subjected to a swirling culture on a rotary shaker (OPTIMA, Inc.) at a speed of 90 rpm along the horizontal plane to draw a circle with a swirling width (diameter) of 25 mm, and cells were cultured in suspension under a condition at 5%CO₂ and 37°C. At the next day after the start of culture (Day 1 of culture), images were obtained by phase contrast microscopy. A control test was conducted using a cell suspension prepared under the same condition as above except that Y-27632 and TRAP-6 were not added.

### (Results)

Figure 1 shows micrographs after the suspension culture described above (Day 1 of culture). As a result of the observation, no aggregates were formed in the control study (0 µM Y-27632, 0 µM TRAP-6) and cells remained in a single cell, while aggregates were formed when Y-27632 was added. Meanwhile, aggregates were formed under conditions in which Y-27632 and TRAP-6 (final concentration 2 µM, 10 µM or 50 µM) were added, but the aggregation was suppressed and smaller aggregates were formed compared to the case in which only Y-27632 was added.

### <Example 3: Effect of Presence of TRAP-6 on Cell Proliferation Potential and Undifferentiated State After Formation of Aggregate>

Suspension culture of human iPS cells was performed, and the glucose consumption, the cell yield, and the percentage of cells positive for undifferentiation markers were determined to analyze the effect of TRAP-6 on cells.

### (Protocol)

A cell suspension was prepared in the same manner as in Example 2, and separately, the cell aggregation suppressor was adjusted so that the final concentration of TRAP-6 (same as above) was 3.74 mg/mL (10 mM). Then the adjusted cell aggregation suppressor was added to the cell suspension so that the final concentration of the agonist of a thrombin receptor was 5 µM. Then the cells were seeded at a ratio of 4 mL/well in a 6-well plate for suspension culture (Sumitomo Bakelite Co., Ltd.). The cell-seeded plate was subjected to a swirling culture on a rotary shaker (OPTIMA, Inc.) at a speed of 75 rpm along the horizontal plane to draw a circle with a swirling width (diameter) of 25 mm, and cells were cultured in suspension under a condition at 5%CO₂ and 37°C. After the next day of culture (Day 1 of culture), the culture medium was exchanged daily for fresh medium (Essential 8™ culture medium containing BSA (Wako Pure Chemical Industries, Ltd.) at a final concentration of 5 mg/mL), and the culture continued until Day 5 of culture. A control test was conducted using a cell suspension prepared under the same condition as described above except that TRAP-6 was not added. Images were obtained by phase contrast microscopy every day during culture. The concentration of glucose contained in the culture supernatant collected at the time of culture medium exchange was measured with biosensor BF-5iD (Prince Measuring Equipment Co., Ltd.) to calculate glucose consumption.

In addition, at Day 5 of culture, cell aggregates were collected, dispersed with Accutase, and then suspended in Essential 8™ culture medium containing 5 mg/mL BSA. A portion of this cell suspension was stained with trypan blue and the number of cells was counted. After the above cell suspensions were centrifuged at 300 g for 3 minutes, the supernatant was then removed, and the cells were washed with PBS (phosphate buffered saline). Next, the cells were fixed with 4% paraformaldehyde (Wako Pure Chemical Industries, Ltd.) at room temperature for 20 minutes, then washed 3 times with PBS. After cells were resuspended with 300 µL of PBS, 3 mL of cold methanol was added while stirring with voltex, and permeabilized at -20°C overnight or more. After 3 washes with 3% FBS (fetal bovine serum)/PBS, cells were resuspended with 3% FBS (fetal bovine serum)/PBS and blocked at room temperature for 30 minutes to 1 hour. Subsequently, the cells were stained with fluorescently labeled anti-SOX2 antibodies (Cat. No. 656110, BioLegend, Inc.) and fluorescently labeled anti-OCT4 antibodies (Cat. No. 653703, BioLegend, Inc.) and fluorescently labeled anti-Nanog antibodies (Cat. No. 674010, BioLegend, Inc.) at 4°C for 30 minutes to 1 hour. After washed once with 3% FBS (fetal bovine serum)/PBS, the cells were made to pass through a cell strainer. The resulting cells were analyzed on FACSVerse. A control test was conducted using cells that were treated in the same way except for reacting, instead of the above three antibodies (fluorescently labeled anti-SOX2 antibody, fluorescently labeled anti-OCT4 antibody, fluorescently labeled anti-Nanog antibody), with three fluorescently labeled isotype control antibodies (Cat. No. 400129, Cat. No. 400314, Cat. No. 400136; BioLegend, Inc.) corresponding to each of the above three antibodies.

Cell yields were also measured at Day 5 of culture. The following procedure was used to measure the cell yields. Specifically, the cell aggregates that had been formed were treated with Accutase for 5 to 10 minutes, pipetted using a blue tip to monodisperse cells, and stained with trypan blue. After that, the number of cells was counted using a hemocytometer to determine the cell yield.

### (Results)

Figure 2 is micrographs observed from Day 1 to Day 5 of culture. Cell aggregates were formed after seeding to Day 1 of culture, and the addition of TRAP-6 resulted in formation of smaller aggregates. By continuing the culture, cells were gradually proliferated and cell aggregates were expanded.

Glucose consumption is shown in Figure 3, and cell yields at Day 5 of culture are shown in Figure 4, respectively. The glucose consumption under the TRAP-6 added condition was greater than that under the TRAP-6 non-added condition, suggesting that cells were proliferating. It was revealed that the number of seeded cells (8 x 10⁵ cells/well) had proliferated 8.5-fold at Day 5 of culture.

Figure 5 shows the results of measuring the percentage of cells positive for undifferentiation markers. In cells obtained by producing cell aggregates in a culture medium comprising TRAP-6 followed by proliferation in suspension culture, the percentages of cells positive for markers SOX2, OCT4 and Nanog were found to be 99% or more, 98% or more, and 99% or more, respectively. This verified that the human iPS cell aggregates that had been formed by the addition of Y-27632 and TRAP-6 remained undifferentiated.

### <Example 4: Confirmation of Aggregation Suppression Effect by Cell Aggregation Assay 2>

### (Protocol)

Cell suspensions were prepared in the same manner as in Example 2 using human iPS cells cultured with the protocol of Example 1, and instead of TRAP-6, a PAR-1 agonist (Proteinase Activated Receptor-1; PAR-1 agonist, ANASPEC, AS-62937, a C-terminal amidated peptide consisting of the amino acid sequence of TFLLRN (SEQ ID NO: 8)) was added so that a final concentration thereof was 32.8 µM or S1P (sphingosine-1-phosphate; Cayman, 62570) (positive control) was added so that the final concentration thereof was 0.8 µg/mL). Then, suspension culture and image acquisition were performed in the same manner as in Example 2.

### (Results)

Figure 6 shows micrographs after the suspension culture described above (Day 1 of culture). As a result of the observation, no aggregates were formed in the control study (0 µM Y-27632, 0 µM PAR-1 agonist) and cells remained in a single cell (data not shown), while aggregates were formed when Y-27632 was added (Figure 6, negative control). Meanwhile, aggregates were formed under conditions in which the PAR-1 agonist (final concentration 32.8 µM) or S1P (0.8 µg/mL, positive control) was added in addition to Y-27632, but the aggregation was suppressed and smaller aggregates were formed compared to the case in which only Y-27632 was added.

### <Example 5: Quantitative RT-PCR Analysis>

Expression of PAR genes (PAR1 and PAR2) in human iPS cells (TkDN4-M cell line, 201B7 cell line, RPChiPS771-2 cell line) was examined.

### (Protocol)

TkDN4-M cell lines (Institute of Medical Science, The University of Tokyo), 201B7 cell lines (Kyoto University), or RPChiPS771-2 cell lines (ReproCELL Incorporated) were used as human iPS cells. These human iPS cells were cultured according to the method described in Example 1. The total RNA was then isolated and purified with TRIzol (Thermo Fisher Scientific Inc.) and PureLink™ RNA Mini Kit (Thermo Fisher Scientific Inc.), and cDNA synthesis was performed with ReverTra Ace™ qPCR RT Master Mix (TOYOBO CO., LTD.). Using the synthesized cDNA as a template, quantitative RT-PCR analysis was performed by QuantStadio 7 Flex Real-Time PCR System (Thermo Fisher Scientific Inc.) with KOD SYBR™ qPCR Mix (TOYOBO CO., LTD.). Detection was performed by SYBR Green intercalation method, and comparison of gene expression amounts was performed by relative quantification method by comparison of ΔCt values using a housekeeping gene (β-actin) as an internal standard. SOX2, an undifferentiation marker gene of pluripotent stem cells, was used as a positive control for gene expression.

The nucleotide sequences of primers used for quantitative RT-PCR are as follows:
β-Actin-F: CCTCATGAAGATCCTCACCGA (SEQ ID NO: 15)
β-Actin-R: TTGCCAATGGTGATGACCTGG (SEQ ID NO: 16)
PAR1-F: GAAGTCCCGGGCTTTGTTCC (SEQ ID NO: 17)
PAR1-R: TGGCACTCAGAGGAAGCGTAA (SEQ ID NO: 18)
PAR2-F: GGCCCTCAGAGATGATCAGTC (SEQ ID NO: 19)
PAR2-R: GTCTCGAACTCCTGACCTCAAG (SEQ ID NO: 20)
SOX2-F: CACCAATCCCATCCACACTCAC (SEQ ID NO: 21)
SOX2-R: GCAAAGCTCCTACCGTACCAC (SEQ ID NO: 22)

### (Results)

The results of the quantitative RT-PCR described above are shown in Figures 7 and 8. In human iPS cells (TkDN4M cell line), PAR1 and PAR2 were expressed at levels similar to the undifferentiation marker gene SOX2 (Figure 7). It was also revealed that PAR1 and PAR2 were expressed in all three human iPS cells used (Figure 8).

### <Example 6: Size Distribution of Cell Aggregates>

### (Protocol)

For cells cultured in suspension in the presence of TRAP-6 according to Example 3, 210 cell aggregates in an image acquired at Day 1 of culture were observed and compared using a micrograph scale, the width of the widest portion (referred to as "φ") of each cell aggregate was measured, then distribution thereof was examined, and the average ± standard deviation was calculated.

### (Results)

Figure 9 is a distribution diagram of cell aggregate size (diameter) at Day 1 of culture. Table 1 below shows the number and percentage of cell aggregates by size at Day 1 of culture.

The result of calculation of an average ± standard deviation of cell aggregate size (diameter) at Day 1 of culture was 167.8 ± 35.3 µm.

Furthermore, the percentage of cell aggregates whose cell aggregate size (diameter) was 40 µm or more and 300 µm or less was 100% to the total cell aggregate count. The percentage of cell aggregates whose cell aggregate size (diameter) was 60 µm or more and 300 µm or less was 100%. The percentage of cell aggregates whose cell aggregate size (diameter) was 80 µm or more and 300 µm or less was 98.0%. The percentage of cell aggregates whose cell aggregate size (diameter) was 100 µm or more and 300 µm or less was 97.0%.

### [Table 1]

**Table 1. Number and percentage of cell aggregates by size**

| Size range (µm) | 40- | 60- | 80- | 100- | 120- | 140- | 160- | 180- | 200- | 220- | 240- | 260- | 280- | 300- |
|---|---|---|---|---|---|---|---|---|---|---|---|---|---|---|
| Number | 0 | 4 | 2 | 5 | 25 | 58 | 56 | 31 | 13 | 8 | 3 | 4 | 1 | 0 |
| Percentage | 0% | 2% | 1% | 2% | 12% | 28% | 27% | 15% | 6% | 4% | 1% | 2% | 0% | 0% |

All the publications, patents, and patent applications cited herein are incorporated herein by reference in its entirety.

## Claims

1. A cell aggregation suppressor for use in suspension culture of cells, comprising an agonist of a thrombin receptor.

2. The cell aggregation suppressor according to claim 1, wherein a concentration of the agonist of a thrombin receptor is 7.4 µg/mL or more and 3.8 mg/mL or less.

3. The cell aggregation suppressor according to claim 1 or 2, wherein the thrombin receptor is at least one selected from the group consisting of PAR-1, PAR-2, PAR-3, and PAR-4.

4. The cell aggregation suppressor according to any one of claims 1 to 3, wherein the agonist of a thrombin receptor is any one of peptides (a), (b), and (c):
(a) a peptide consisting of an amino acid sequence of SEQ ID NO: 1, 2, 7, or 8;
(b) a peptide consisting of an amino acid sequence derived from the amino acid sequence of SEQ ID NO: 1, 2, 7, or 8 by substitution, deletion, insertion, and/or addition of one or two amino acids;
(c) a peptide consisting of an amino acid sequence having 60% or more sequence identity to the amino acid sequence of SEQ ID NO: 1, 2, 7, or 8.

5. The cell aggregation suppressor according to any one of claims 1 to 4, wherein the cells are stem cells.

6. A method for producing cell aggregates, comprising a step of culturing cells in suspension in a culture medium comprising an agonist of a thrombin receptor.

7. The method according to claim 6, wherein a concentration of the agonist of a thrombin receptor in the culture medium is 3.7 ng/mL or more and 3.8 mg/mL or less.

8. The method according to claim 6 or 7, wherein the thrombin receptor is at least one selected from the group consisting of PAR-1, PAR-2, PAR-3, and PAR-4.

9. The method according to any one of claims 6 to 8, wherein the agonist of a thrombin receptor is any one of peptides (a), (b), and (c):
(a) a peptide consisting of an amino acid sequence of SEQ ID NO: 1, 2, 7, or 8;
(b) a peptide consisting of an amino acid sequence derived from the amino acid sequence of SEQ ID NO: 1, 2, 7, or 8 by substitution, deletion, insertion, and/or addition of one or two amino acids;
(c) a peptide consisting of an amino acid sequence having 60% or more sequence identity to the amino acid sequence of SEQ ID NO: 1, 2, 7, or 8.

10. The method according to any one of claims 6 to 9, wherein the cells are stem cells.

11. A cell aggregate obtained by the method according to any one of claims 6 to 10.

12. A cell culture composition comprising cells, a culture medium, and an agonist of a thrombin receptor.

13. The cell culture composition according to claim 12, wherein a concentration of the agonist of a thrombin receptor is 3.7 ng/mL or more and 3.8 mg/mL or less.

14. The cell culture composition according to claim 12 or 13, wherein the thrombin receptor is at least one selected from the group consisting of PAR-1, PAR-2, PAR-3, and PAR-4.

15. The cell culture composition according to any one of claims 12 to 14, wherein the agonist of a thrombin receptor is any one of peptides (a), (b), and (c):
(a) a peptide consisting of an amino acid sequence of SEQ ID NO: 1, 2, 7, or 8;
(b) a peptide consisting of an amino acid sequence derived from the amino acid sequence of SEQ ID NO: 1, 2, 7, or 8 by substitution, deletion, insertion, and/or addition of one or two amino acids;
(c) a peptide consisting of an amino acid sequence having 60% or more sequence identity to the amino acid sequence of SEQ ID NO: 1, 2, 7, or 8.

16. The cell culture composition according to any one of claims 12 to 15, wherein the cells are stem cells.

17. The cell culture composition according to any one of claims 12 to 16, wherein the cells are in a form of cell aggregates.
